(19) Europäisches Patentamt
European Patent Office
Office européen des brevets

(11) EP 1 664 055 B1

# (12) FASCICULE DE BREVET EUROPEEN

(45) Date de publication et mention de la délivrance du brevet:
**13.12.2006 Bulletin 2006/50**

(21) Numéro de dépôt: **04787274.2**

(22) Date de dépôt: **01.09.2004**

(51) Int Cl.:
***C07D 493/04*** (2006.01) ***A61K 31/36*** (2006.01)
***A61P 35/00*** (2006.01)

(86) Numéro de dépôt international:
**PCT/FR2004/002218**

(87) Numéro de publication internationale:
**WO 2005/023817 (17.03.2005 Gazette 2005/11)**

(54) **DERIVES DE 9-AMINO-PODOPHYLLOTOXINE, LEUR PROCEDE DE PREPARATION ET LES COMPOSITIONS PHARMACEUTIQUES QUI LES CONTIENNENT**

9-AMINOPODOPHYLLOTOXINDERIVATE, VERFAHREN ZU DEREN HERSTELLUNG UND PHARMAZEUTISCHE ZUSAMMENSETZUNGEN, DIE DIESE DERIVATE ENTHALTEN

9-AMINO-PODOPHYLLOTOXIN DERIVATIVES, METHOD FOR THE PRODUCTION THEREOF AND PHARMACEUTICAL COMPOSITIONS CONTAINING SAID DERIVATIVES

(84) Etats contractants désignés:
**CH DE FR GB LI**

(30) Priorité: **02.09.2003 FR 0310367**

(43) Date de publication de la demande:
**07.06.2006 Bulletin 2006/23**

(73) Titulaires:
- **Les Laboratoires Servier**
  **92415 Courbevoie Cédex (FR)**
- **CENTRE NATIONAL DE LA RECHERCHE SCIENTIFIQUE (CNRS)**
  **75794 Paris Cedex 16 (FR)**

(72) Inventeurs:
- **MONNERET, Claude**
  **F-75012 Paris (FR)**
- **DAUZONNE, Daniel**
  **F-75011 Paris (FR)**
- **HICKMAN, John**
  **F-75017 Paris (FR)**
- **PIERRE, Alain**
  **F-78580 les Alluets le Roi (FR)**
- **KRAUS-BERTHIER, Laurence**
  **F-92700 Colombes (FR)**
- **PFEIFFER, Bruno**
  **F-95320 Saint Leu la Foret (FR)**
- **RENARD, Pierre**
  **F-78150 Le Chesnay (FR)**

(74) Mandataire: **Warcoin, Jacques et al**
**Cabinet Régimbeau**
**20, rue de Chazelles**
**75847 Paris cedex 17 (FR)**

(56) Documents cités:
**WO-A-90/09788** **US-A- 5 536 847**
**US-A- 5 541 223**

- **CHO SUNG JIN ET AL: "Antitumor Agents. 163. Three-dimensional quantitative structure-activity relationship study of 4'-O-demethylepipodophyllotoxin analogs using the CoMFA/q2-GRS approach" JOURNAL OF MEDICINAL CHEMISTRY, AMERICAN CHEMICAL SOCIETY. WASHINGTON, US, vol. 39, 1996, pages 1383-1395, XP002222563 ISSN: 0022-2623**

EP 1 664 055 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention concerne de nouveaux dérivés de 9-amino-podophyllotoxine, leur procédé de préparation et les compositions pharmaceutiques qui les contiennent.

Les composés de l'invention constituent des dérivés de la podophyllotoxine, un lignane naturel connu pour son utilité dans le traitement du cancer. D'autres dérivés synthétiques tels que l'étoposide et la téniposide sont utilisés de façon courante comme agents chimiothérapeutique pour le traitement notamment du cancer du poumon à petites cellules. Ces différents composés agissent en inhibant l'activité catalytique de la topoisomérase II en stabilisant le complexe clivable.

Diverses modifications ont été réalisées sur ces dérivés, comme celles décrites dans les demandes de brevet JP 948782, WO 97/13776 et US 3,634,459. Néanmoins, les besoins de la thérapeutique anticancéreuse exigent le développement constant de nouveaux agents antitumoraux et cytotoxiques, dans le but d'obtenir des médicaments à la fois plus actifs, plus solubles et mieux tolérés.

Les composés de la présente invention, outre le fait qu'ils soient nouveaux, présentent une activité *in vivo* et *in vitro* surprenante et supérieure à celle observée jusqu'ici. Ainsi, les composés de la présente invention possèdent des propriétés qui les rendent particulièrement utiles pour le traitement des cancers. Parmi les types de cancers qui peuvent être traités par les composés de la présente invention, on peut citer à titre non limitatif les adénocarcinomes et carcinomes, sarcomes, gliomes et leucémies.

Plus particulièrement, la présente invention concerne les composés de formule (I) :

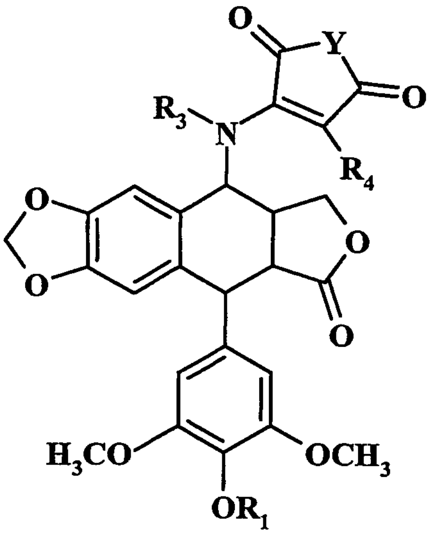

(I)

dans laquelle :

$R_1$ représente un groupement choisi parmi hydrogène, alkyle ($C_1$-$C_6$) linéaire ou ramifié, aryle, arylalkyle ($C_1$-$C_6$) linéaire ou ramifié, hétéroaryle, hétéroarylalkyle ($C_1$-$C_6$) linéaire ou ramifié, alkylcarbonyle ($C_1$-$C_6$) linéaire ou ramifié, arylcarbonyle, arylalkylcarbonyle ($C_1$-$C_6$) linéaire ou ramifié, alkoxycarbonyle ($C_1$-$C_6$) linéaire ou ramifié, aryloxycarbonyle, arylalkoxycarbonyle ($C_1$-$C_6$) linéaire ou ramifié, hétérocycloalkoxycarbonyle, alkylsulfonyle ($C_1$-$C_6$) linéaire ou ramifié, arylsulfonyle, arylalkylsulfonyle ($C_1$-$C_6$) linéaire ou ramifié, phosphonique, ou $Si(R_a)_2R_b$ dans laquelle $R_a$, $R_b$, identiques ou différents, représentent chacun un groupement choisi parmi alkyle ($C_1$-$C_6$) linéaire ou ramifié ou aryle,

Y représente un groupement choisi parmi HN-NH ou N-$R_2$ dans laquelle :

$R_2$ représente un groupement choisi parmi hydrogène, alkyle ($C_1$-$C_6$) linéaire ou ramifié, aryle, hétéroaryle, cycloalkyle, hétérocycloalkyle, alkényle ($C_2$-$C_6$) linéaire ou ramifié, alkynyle ($C_2$-$C_6$) linéaire ou ramifié ou un groupement de formule -$T_1$-$R_5$ dans laquelle :

- ♦ $T_1$ représente un groupement choisi parmi une chaîne alkylène ($C_1$-$C_6$) linéaire ou ramifiée éventuellement substituée par un ou plusieurs groupements choisi parmi hydroxy ou alkoxy ($C_1$-$C_6$) linéaire ou ramifié, une chaîne

alkénylène ($C_2$-$C_6$) linéaire ou ramifiée ou une chaîne alkynylène ($C_2$-$C_6$) linéaire ou ramifiée,

♦ $R_5$ représente un groupement choisi parmi hydroxy, alkoxy ($C_1$-$C_6$) linéaire ou ramifié, alkylcarbonyle ($C_1$-$C_6$) linéaire ou ramifié, alkylcarbonyloxy ($C_1$-$C_6$) linéaire ou ramifié, alkoxycarbonyle ($C_1$-$C_6$) linéaire ou ramifié, carboxy, halogène, trihalogénométhyle, aryle, hétéroaryle, cycloalkyle, hétérocycloalkyle, $NR_cR_d$ dans lequel $R_c$, $R_d$, identiques ou différents, représentent chacun un groupement choisi parmi hydrogène, alkyle ($C_1$-$C_6$) linéaire ou ramifié, aminoalkyle ($C_1$-$C_6$) linéaire ou ramifié dans lequel la partie amino est éventuellement substituée par un ou deux groupements identiques ou différents, alkyle ($C_1$-$C_6$) linéaire ou ramifié, hydroxyalkyle ($C_1$-$C_6$) linéaire ou ramifié, alkoxy ($C_1$-$C_6$) alkyle ($C_1$-$C_6$) linéaire ou ramifié
ou $C(O)NR'_cR'_d$ dans lequel $R'_c$, $R'_d$, identiques ou différents, représentent chacun un groupement choisi parmi hydrogène, alkyle ($C_1$-$C_6$) linéaire ou ramifié, aminoalkyle ($C_1$-$C_6$) linéaire ou ramifié dans lequel la partie amino est éventuellement substituée par un ou deux groupements identiques ou différents, alkyle ($C_1$-$C_6$) linéaire ou ramifié, hydroxyalkyle ($C_1$-$C_6$) linéaire ou ramifié, alkoxy ($C_1$-$C_6$) alkyle ($C_1$-$C_6$) linéaire ou ramifié ou $R'_c$ et $R'_d$ forment ensemble avec l'atome d'azote qui les porte, un hétérocycloalkyle,

$R_3$ représente un groupement choisi parmi hydrogène, alkyle ($C_1$-$C_6$) linéaire ou ramifié, cycloalkyle, cycloalkylalkyle ($C_1$-$C_6$) linéaire ou ramifié, aryle ou arylalkyle ($C_1$-$C_6$) linéaire ou ramifié,

$R_4$ représente un groupement choisi parmi hydrogène, alkyle ($C_1$-$C_6$) linéaire ou ramifié,

leurs énantiomères, diastéréoisomères ainsi que leurs sels d'addition à un acide ou à une base pharmaceutiquement acceptable,
étant entendu que :

* *par aryle,* on comprend un groupement choisi parmi phényle, biphényle, naphtyle, dihydronaphtyle, tétrahydronaphtyle, indényle, indanyle, et benzocyclobutyle, chacun de ces groupements comportant éventuellement une ou plusieurs substitutions, identiques ou différentes, choisies parmi halogène, hydroxy, alkyle ($C_1$-$C_6$) linéaire ou ramifié, alkoxy ($C_1$-$C_6$) linéaire ou ramifié, cyano, nitro, amino, alkylamino ($C_1$-$C_6$) linéaire ou ramifié, dialkylamino ($C_1$-$C_6$) linéaire ou ramifié, carboxy, alkoxycarbonyle ($C_1$-$C_6$) linéaire ou ramifié, trihalogénoalkyle ($C_1$-$C_6$) linéaire ou ramifié, alkylcarbonyloxy ($C_1$-$C_6$) linéaire ou ramifié, alkylcarbonyle ($C_1$-$C_6$) linéaire ou ramifié, et aminocarbonyle dans laquelle la partie amino est éventuellement substituée par un ou deux groupements, identiques ou différents, alkyle ($C_1$-$C_6$) linéaire ou ramifié,

* *par hétéroaryle,* on comprend un groupement mono- ou bicyclique aromatique ou un groupement bicyclique dont l'un des cycles est aromatique et l'autre cycle est partiellement hydrogéné, de 5 à 12 chaînons, comportant au sein du système cyclique de un à trois hétéroatomes, identiques ou différents, choisis parmi oxygène, azote et soufre, ledit groupement hétéroaryle pouvant éventuellement être substitué par un ou plusieurs groupements identiques ou différents, choisis parmi les substituants définis précédemment dans le cas du groupement aryle ; parmi les groupements hétéroaryles, on peut citer à titre non limitatif le groupement pyridyle, pyrrolyle, thiényle, furyle, pyrazinyle, isothiazolyle, thiazolyle, oxazolyle, isoxazolyle, pyrimidinyle, indolyle, benzofuranyle, benzothiényle, quinolyle, isoquinolyle, benzo[1,4]dioxynyle, 2,3-dihydrobenzo[1,4]dioxynyle,

* *par cycloalkyle,* on comprend un groupement mono- ou bicyclique, saturé ou insaturé mais sans caractère aromatique, comportant de 3 à 12 atomes de carbone, étant éventuellement substitué par un ou plusieurs groupements, identiques ou différents, choisis parmi halogène, alkyle ($C_1$-$C_6$) linéaire ou ramifié, trihalogénoalkyle ($C_1$-$C_6$) linéaire ou ramifié, hydroxy, amino, alkylamino ($C_1$-$C_6$) linéaire ou ramifié et dialkylamino ($C_1$-$C_6$) linéaire ou ramifié ; parmi les groupements cycloalkyles, on peut citer à titre non limitatif, le groupement cyclopropyle, cyclobutyle, cyclopentyle, cyclohexyle,

* *par hétérocycloalkyle,* on comprend un cycloalkyle tel que défini précédemment, comportant au sein du système cyclique, de un à deux hétéroatomes, identiques ou différents, choisis parmi oxygène et azote, ledit hétérocycloalkyle étant éventuellement substitué par un ou plusieurs groupements identiques ou différents définis précédemment dans le cas du groupement cycloalkyle ; parmi les groupements hétérocycloalkyles, on peut citer à titre non limitatif le groupement pipéridyle, pipérazinyle, morpholyle.

Parmi les acides pharmaceutiquement acceptables, on peut citer à titre non limitatif, les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium,

l'hydroxyde de potassium, la triéthylamine, la *tert*-butylamine, etc...

Le substituant $R_1$ préféré selon l'invention est l'atome d'hydrogène.

Le substituant $R_3$ préféré selon l'invention est l'atome d'hydrogène.

Le substituant $R_4$ préféré selon l'invention sont l'atome d'hydrogène et le groupement méthyle.

D'une façon très avantageuse, les composés préférés de l'invention sont les composés de formule (I) dans laquelle Y représente un groupement HN-NH ou N-$R_2$ dans lequel $R_2$ représente un groupement alkyle ($C_1$-$C_6$) linéaire ou ramifié, alkényle ($C_2$-$C_6$) linéaire ou ramifié ou un groupement de formule -$T_1$-$R_5$ dans laquelle $T_1$ et $R_5$ sont tels que définis dans la formule (I).

D'une façon particulièrement avantageuse, le groupement Y préféré selon l'invention est le groupement de formule $NR_2$ dans lequel $R_2$ représente un groupement méthyle.

D'une façon intéressante, le groupement Y préféré selon l'invention est le groupement de formule $NR_2$ dans lequel $R_2$ représente un groupement $T_1$-$R_5$ dans lequel $T_1$ représente une chaîne alkylène ($C_1$-$C_6$) linéaire ou ramifiée et $R_5$ représente un groupement choisi parmi aryle, carboxy et alkylcarbonyloxy ($C_1$-$C_6$) linéaire ou ramifié.

D'une façon encore plus intéressante, le groupement Y préféré selon l'invention est le groupement de formule $NR_2$ dans lequel $R_2$ représente un groupement $T_1$-$R_5$ dans lequel $T_1$ représente un groupement méthylène -$CH_2$- et $R_5$ représente un groupement aryle.

Les composés préférés de l'invention sont le :

- ♦ 3{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-méthyl-1*H*-pyrrole -2,5-dione ;
- ♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-benzyl-1*H*-pyrrole-2,5-dione ;
- ♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-(4-fluorobenzyl)-1*H*-pyrrole-2,5-dione ;
- ♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-[4-(trifluorométhyl)benzyl]-1*H*-pyrrole-2,5-dione ;
- ♦ *N*-{4-[(3-{[(5*S*,5a*S*,5a*S*,8a*R*,9*R*)-9-(4-hydroxy-3,5-diméthoxyphényl)-8-oxo-5,5a,6,8,8a,9-hexahydrofuro[3',4':6,7]naphtho[2,3-*d*][1,3]dioxol-5-yl]amino}-2,5-dioxo-2,5-dihydro-1*H*-pyrrol-1-yl)méthyl]phényl} acétamide.
- ♦ Acide 6-(3- {[(5*S*,5a*S*,8a*R*,9*R*)-9-(4-hydroxy-3,5-diméthoxyphényl)-8-oxo-5,5a,6,8,8a,9-hexahydrofuro[3',4':6,7] naphtho[2,3-*d*][1,3]dioxol-5-yl]amino}-2,5-dioxo-2,5-dihydro-1*H*-pyrrol-1-yl)hexanoïque
- ♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-butyl-1*H*-pyrrole-2,5-dione
- ♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-allyl-1*H*-pyrrole-2,5-dione
- ♦ Acétate de 2-(3-{[(5*S*,5a*S*,8a*R*,9*R*)-9-(4-hydroxy-3,5-diméthoxyphényl)-8-oxo-5,5a,6,8, 8a,9-hexahydrofuro[3', 4':6,7]naphtho[2,3-*d*][1,3]dioxol-5-yl]amino}-2,5-dioxo-2,5-dihydro-1*H*-pyrrole-1-yl)éthyle
- ♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-(2,3-dihydroxypropyl)-1*H*-pyrrole-2,5-dione
- ♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-[2-(diméthylamino) éthyl]-1*H*-pyrrole-2,5-dione

Les énantiomères, diastéréoisomères ainsi que les sels d'addition à un acide ou à une base pharmaceutiquement acceptable des composés préférés de l'invention font partie intégrante de l'invention.

La présente invention s'étend également au procédé de préparation des composés de formule (I), caractérisé en ce qu'on utilise comme produit de départ, un composé de formule (II) :

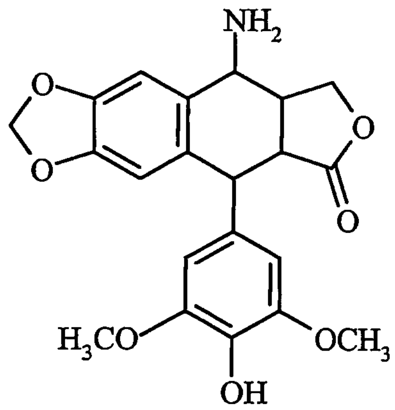

(II)

qui est soumis, en condition basique :

➢ soit à l'action d'un composé de formule (III) :

$R'_1$-X (III)

dans laquelle $R'_1$ représente un groupement choisi parmi alkyle ($C_1$-$C_6$) linéaire ou ramifié, aryle, arylalkyle ($C_1$-$C_6$) linéaire ou ramifié, hétéroaryle, hétéroarylalkyle ($C_1$-$C_6$) linéaire ou ramifié, alkylcarbonyle ($C_1$-$C_6$) linéaire ou ramifié, arylcarbonyle, arylalkylcarbonyle ($C_1$-$C_6$) linéaire ou ramifié, alkoxycarbonyle ($C_1$-$C_6$) linéaire ou ramifié, aryloxycarbonyle, arylalkoxycarbonyle ($C_1$-$C_6$) linéaire ou ramifié, hétérocycloalkoxycarbonyle, alkylsulfonyle ($C_1$-$C_6$) linéaire ou ramifié, arylsulfonyle, arylalkylsulfonyle ($C_1$-$C_6$) linéaire ou ramifié, phosphonique, ou $Si(R_a)_2R_b$ dans laquelle $R_a$, $R_b$, identiques ou différents, représentent chacun un groupement choisi parmi alkyle ($C_1$-$C_6$) linéaire ou ramifié ou aryle,
et X représente un atome d'hydrogène, un atome d'halogène ou un groupe partant usuel de la chimie organique, pour conduire aux composés de formule (IV/a) :

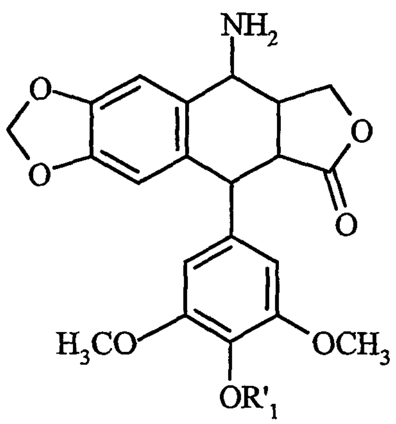

(IV/a)

dans laquelle $R'_1$ est tel que défini précédemment,

➢ soit à l'action d'un composé de formule (V) :

G-L (V)

dans laquelle G représente un groupement protecteur classique des fonctions hydroxy et L un groupe partant usuel de la chimie organique, pour conduire aux composés de formule (IV/b) :

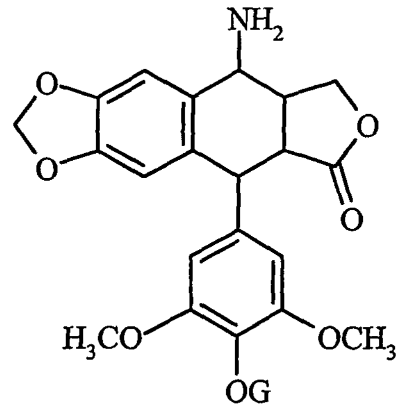

(IV/b)

dans laquelle G est tel que défini précédemment,
l'ensemble des composés de formule (IV/a) et (IV/b) formant les composés de formule (IV):

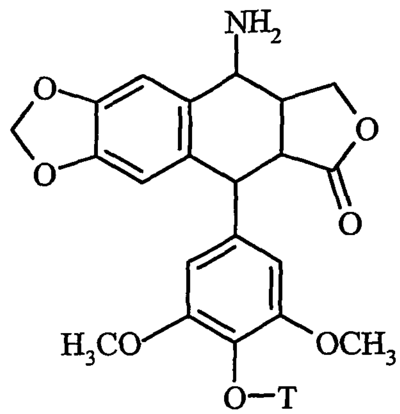

(IV)

dans laquelle T représente un groupement $R'_1$ ou G tels que définis précédemment,
composé de formule (IV), qui est soumis, en condition basique, à l'action d'un composé de formule (VI) :

$R'_3$-X' (VI)

dans laquelle $R'_3$ représente un groupement choisi parmi alkyle ($C_1$-$C_6$) linéaire ou ramifié, cycloalkyle, cycloalkylalkyle ($C_1$-$C_6$) linéaire ou ramifié, aryle ou arylalkyle ($C_1$-$C_6$) linéaire ou ramifié,
et X' représente un atome d'hydrogène, un atome d'halogène ou un groupe partant usuel de la chimie organique, pour conduire aux composés de formule (VII) :

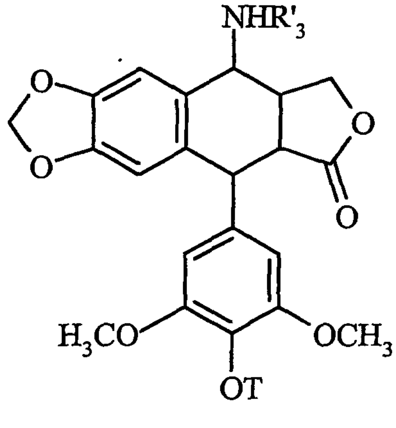

(VII)

dans laquelle $R'_3$ et T sont tels que définis précédemment,
l'ensemble des composés de formules (IV) et (VII) formant les composés de formule (VIII) :

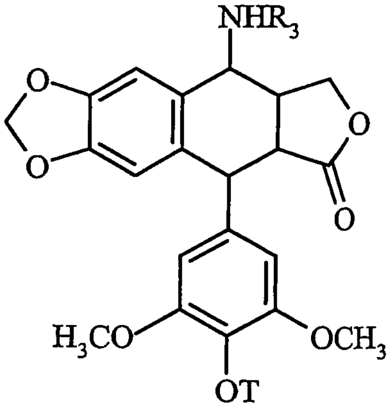

(VIII)

dans laquelle $R_3$ et T sont tels que définis dans la formule (I),
composé de formule (VIII) que l'on traite en milieu basique par un composé de formule (IX):

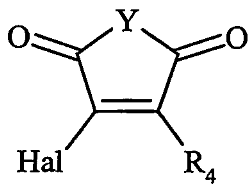

(IX)

dans laquelle Y et $R_4$ sont tels que définis dans la formule (I),
et Hal représente un atome d'halogène, pour conduire aux composés de formules (I/a) et (I/b), cas particuliers des composés de formule (I), selon que T représente un groupement $R'_1$ ou G respectivement :

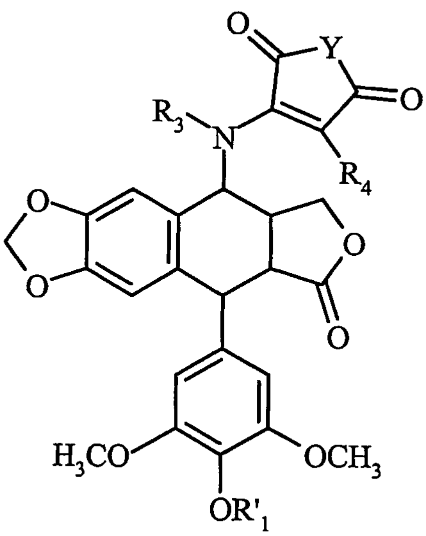

(I/a)

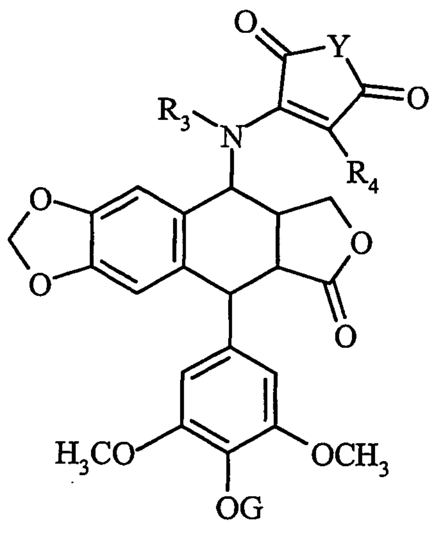

(I/b)

dans laquelle $R'_1$, $R_3$, $R_4$, Y et G sont tels que définis précédemment,
composé de formule (I/b) dont on déprotège la fonction hydroxy selon les méthodes classiques de la chimie organique, pour conduire aux composés de formule (I/c), cas particuliers des composés de formule (I) :

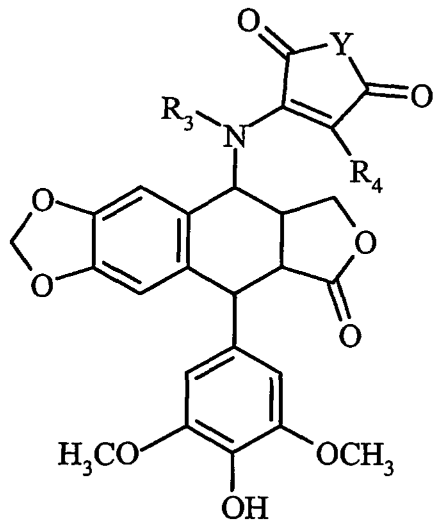

(I/c)

dans laquelle $R_3$, $R_4$ et Y sont tels que définis précédemment,
les composés (I/a) à (I/c) formant l'ensemble des composés de l'invention, que l'on purifie, le cas échéant, selon une technique classique de purification, qui peuvent, si on le désire, être séparés en leurs différents isomères optiques selon une technique classique de séparation, et que l'on transforme, si on le souhaite, en leurs sels d'addition à un acide ou à une base pharmaceutiquement acceptable.
Les composés de formule (II), (III), (V), (VI) et (IX) sont soit des composés commerciaux, soit obtenus selon les méthodes classiques de synthèse organique.

Les composés de formule (I) présentent des propriétés antitumorales particulièrement intéressantes. Ils ont une excellente cytotoxicité *in vitro* sur des lignées cellulaires, issues de tumeurs murines et humaines, et sont actifs *in vivo.* Les propriétés caractéristiques de ces composés permettent leur utilisation en thérapeutique en tant qu'agents antitumoraux.

La présente invention a également pour obj et les compositions pharmaceutiques contenant comme principe actif au moins un composé de formule (I), ses isomères optiques, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions selon l'invention, il sera cité plus particulièrement celles qui conviennent pour l'administration orale, parentérale (intraveineuse, intramusculaire ou sous-cutanée), per- ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection, et la prise de traitements éventuels associés et s'échelonne de 0,5 mg à 500 mg en une ou plusieurs prises par jour.

Les exemples suivants illustrent l'invention mais ne la limitent en aucune façon. Les produits de départ utilisés sont des produits connus ou préparés selon des modes opératoires connus.

**PREPARATION 1 :**

**(5R,5aR,8aS,9S)-9-amino-5-(4-{[*tert*-butyl(diméthyl)silyl]oxy}-3,5-diméthoxyphényl)-5,8,8a,9-tétrahydrofuro [3',4':6,7]naphtho[2,3-*d*][1,3]dioxol-6(5a*H*)-one**

Une solution de 63,4 mmol de (5R,5aR,8aS,9S)-9-amino-5-(4-hydroxy-3,5-diméthoxyphényl)-5,8,8a,9-tétrahydrofuro[3',4':6,7]naphtho[2,3-*d*][1,3]dioxol-6(5a*H*)-one, 111,8 mmol de chlorure de *tert*-butyldiméthylsilyle et 510 mmol d'imidazole dans 1,6 1 de diméthylformamide anhydre est agitée pendant 20 heures à température ambiante. Le mélange réactionnel est ensuite lavé avec 2 1 d'eau puis 1 1 d'éther. La phase éthérée est alors décantée et la phase aqueuse extraite avec de l'éther. Les phases organiques sont ensuite séchées sur sulfate de magnésium, filtrées et concentrées sous pression réduite. Le produit attendu est obtenu par recristallisation dans un mélange benzène/heptane.

**Point de fusion : 236-238°C**
**Spectrométrie de masse (IC/$NH_3$) : m/z = 514 $[M+H]^+$, 531 $[M+NH_4]^+$**

**PREPARATION 2 :**

**3-Bromo-1-méthyl-1*H*-pyrrole-2,5-dione**

Une solution de 15 mmol de 3-bromo-2,5-furanedione et de 15 mmol de méthylamine à 40 % dans l'eau, dans 300 ml d'acide acétique glacial est porté sous reflux pendant 16 heures. Après avoir laissé revenir le mélange réactionnel à température ambiante, 20 ml d'anhydride acétique sont ajoutés. Le milieu réactionnel est à nouveau porté au reflux sous agitation pendant 4 heures avant d'évaporer les solvants sous pression réduite. Le produit attendu est obtenu après purification par chromatographie sur gel de silice (heptane/acétate d'éthyle : 1/1) et recristallisation dans l'éthanol.
**Point de fusion : 88-89°C**
**Spectrométrie de masse (IC/$NH_3$) : m/z = 207,209 $[M+NH_4]^+$**

**PREPARATION 3 :**

**3-Bromo-1-benzyl-1*H*-pyrrole-2,5-dione**

A une solution de 87,3 mmol de benzylamine dans 345 ml d'acide acétique glacial sont ajoutées 87,3 mmol de 3-bromo-2,5-furanedione. L'ensemble est porté sous reflux pendant 16 heures sous atmosphère d'argon. Le mélange réactionnel est évaporé sous pression réduite après retour à température ambiante. Le résidu obtenu est repris avec 310 ml d'acide acétique auquel sont ajoutés 62,3 mmol d'acétate de sodium. L'ensemble est à nouveau porté sous reflux pendant 2 heures. Après retour à température ambiante, le mélange réactionnel est lavé avec 1 l d'eau et 500 ml d'éther. La phase aqueuse est ensuite extraite avec de l'éther. Les phases organises sont lavées avec une solution saturée de chlorure de sodium (2 x 300 ml), de l'eau (2 x 500 ml), séchées sur sulfate de magnésium, filtrées et concentrées sous pression réduite. Une chromatographie sur gel de silice (dichlorométhane) permet d'isoler le produit attendu.
**Spectrométrie de masse (IC/$NH_3$) : m/z = 283,29 $[M+NH_4]^+$**

**PREPARATION 4 :**

**3-Bromo-1-(4-fluorabenzyl)-1*H*-pyrrole-2,5-dione**

Ce produit est obtenu selon le procédé de la préparation 3 en utilisant la 4-fluorobenzylamine à la place de la benzylamine.

**PREPARATION 5 :**

**3-Bromo-1-[(4-trifluorométhyl)benzyl]-1*H*-pyrrole-2,5-dione**

Ce produit est obtenu selon le procédé de la préparation 3 en utilisant la 4-trifluorométhylbenzylamine à la place de la benzylamine.

**PREPARATION 6 :**

**3-Bromo-1-[(4-acétamido)benzyl]-1*H*-pyrrole-2,5-dione**

Ce produit est obtenu selon le procédé de la préparation 3 en utilisant la 4-acétamidobenzylamine à la place de la benzylamine.

**PREPARATION 7:**

**3-Bromo-1-[2-hydraxy-1-(hydroxyméthyl)éthyl]-1*H*-pyrrole-2,5-dione**

Ce produit est obtenu selon le procédé de la préparation 3 en utilisant le 2-amino-1,3-propanediol à la place de la benzylamine.

**PREPARATION 8 :**

**Acide (3-bromo-2,5-dioxo-2,5-dihydro-1*H*-pyrrol-1-yl)acétique**

Ce produit est obtenu selon le procédé de la préparation 3 en utilisant la glycine à la place de la benzylamine.
**Point de fusion : 153-155°C**
**Spectrométrie de masse (IC/$NH_3$) : m/z = 251,25 [M+$NH_4$]$^+$**

**PREPARATION 9 :**

**Acétate de (3-bromo-2,5-dioxo-2,5-dihydro-1*H*-pyrrol-1-yl)-natêthyle**

Ce produit est obtenu selon le procédé de la préparation 3 en utilisant la glycine méthyl ester à la place de la benzylamine.

**PREPARATION 10 :**

**Acide 6-(3-bromo-2,5-dioxo-2,5-dihydro-1*H*-pyrrol-1-yl)hexanoïque**

Ce produit est obtenu selon le procédé de la préparation 3 en utilisant l'acide 6-aminohexanoïque à la place de la benzylamine.
**Point de fusion : 98-100°C**
**Spectrométrie de masse (IC/$NH_3$) : m/z = 307,31 [M+$NH_4$]$^+$**

**PREPARATION 11 :**

**3-Bromo-1-butyl-1*H*-pyrrole-2,5-dione**

Ce produit est obtenu selon le procédé de la préparation 3 en utilisant la butylamine à la place de la benzylamine.
**Spectrométrie de masse (IC/$NH_3$): m/z = 249,25 [M+$NH_4$]$^+$**

**PREPARATION 12 :**

**(5*R*,5a*R*,8a*S*,9*S*)-9-Amino-5-(3,4,5-triméthoxyphényl)-5,8,8a,9-tétrahydrofuro [3',4':6,7]-naphtho[2,3-*d*][1,3] dioxol-6(5a*H*)-one**

Ce produit est obtenu selon le procédé de la préparation 1 en utilisant l'iodométhane à la place du chlorure de *tert*-butyldiméthylsilyle.

**PREPARATION 13 :**

**3-Bromo-1,4-diméthyl-1*H*-pyrrole-2,5-dione**

Ce produit est obtenu selon le procédé de la préparation 2 en utilisant le 3-bromo-4-méthyl-2,5-furanedione à la place du 3-bromo-2,5-furanedione.

**PREPARATION 14 :**

**(5*R*,5a*R*,8a*S*,9*S*)-5-(4-{[*tert*-Butyl(diméthyl)silyl]oxy}-3,5-diméthoxyphényl)-9-(méthylamino)-5,8,8a,9-tetrahydrofuro[3',4':6,7]naphtho[2,3-*d*][1,3]dioxol-6(5a*H*)-**one

Ce produit est obtenu selon le procédé de l'exemple 1 en utilisant l'iodométhane à la place du composé de la préparation 2.

**PREPARATION 15 :**

**3-Bromo-1-(2-hydroxyéthyl)-1*H*-pyrrole-2,5-dione**

Ce produit est obtenu selon le procédé de la préparation 3 en utilisant l'éthanolamine à la place de la benzylamine.

**PREPARATION 16 :**

**3-Bromo-1-(2-méthoxyéthyl)-1*H*-pyrrole-2,5-dione**

Ce produit est obtenu selon le procédé de la préparation 3 en utilisant la 2-méthoxyéthylamine à la place de la benzylamine.

**PREPARATION 17 :**

**1-Allyl-3-bromo-1*H*-pyrrole-2,5-dione**

Ce produit est obtenu selon le procédé de la préparation 3 en utilisant l'allylamine à la place de la benzylamine.

**PREPARATION 18 :**

**3-Bromo-1-[2-(1-pigéridinyl)éthyl]-1*H*-pyrrole-2,5-dione**

Ce produit est obtenu selon le procédé de la préparation 3 en utilisant la 2-(1-pipéridinyl)éthylamine à la place de la benzylamine.

**PREPARATION 19 :**

**3-Bromo-1-[2-(4-morpholinyl)éthyl]-1*H*-pyrrole-2,5-dione**

Ce produit est obtenu selon le procédé de la préparation 3 en utilisant la 2-(4-morpholinyl)éthylamine à la place de la benzylamine.

**PREPARATION 20 :**

**4-Bromo-1,2-dihydro-3,6-pyridazinedione**

Ce produit est obtenu selon le procédé de la préparation 3 en utilisant l'hydrate d'hydrazine à la place de la benzylamine.
**Point de fusion : 274-276°C**
**Spectrométrie de masse (IC/$NH_3$) : m/z = 208,21 $[M+NH_4]^+$**

**PREPARATION 21 :**

**Acétate de 2-(3-bromo-2,5-dioxo-2,5-dihydro-1*H*-pyrrol-1-yl) éthyle**

Ce produit est obtenu selon le procédé de la préparation 3 en utilisant l'acétate de 2-aminoéthyle à la place de la benzylamine.

**PREPARATION 22 :**

**Acide 7-(3-bromo-2,5-dioxo-2,5-dihydro-1*H*-pyrrol-1-yl) heptanoïque**

Ce produit est obtenu selon le procédé de la préparation 3 en utilisant l'acide 7-aminohexanoïque à la place de la benzylamine.

**PREPARATION 23 :**

**3-Bromo-1-(2,3-dihydroxypropyl)-1*H*-pyrrole-2,5-dione**

Ce produit est obtenu selon le procédé de la préparation 3 en utilisant le 3-amino-1,2-propanediol à la place de la benzylamine.

**PREPARATION 24 :**

**3-Bromo-1-[2-(4-méthyl-1-pipérazinyl)éthyl]-1*H*-pyrrole-2,5-dione**

Ce produit est obtenu selon le procédé de la préparation 3 en utilisant la 2-(4-méthyl-1-pipérazinyl)éthylamine à la place de la benzylamine.

**PREPARATION 25 :**

**3-Bromo-1-[2-(diméthylamino)éthyl-1*H*-pyrrole-2,5-dione**

Ce produit est obtenu selon le procédé de la préparation 3 en utilisant la N-(2-aminoéthyl)-N,N-diméthylamine à la place de la benzylamine.

**PREPARATION 26 :**

**3-Bromo-1-[2-(butylamino)éthyl]-1*H*-pyrrole-2,5-dione**

Ce produit est obtenu selon le procédé de la préparation 3 en utilisant la N-(2-aminoéthyl)-N-butylamine à la place de la benzylamine.

**PREPARATION 27:**

**3-Bromo-1-[2-{[2-(diméthylamino)éthyl]amino}eihyl)-1*H*-pyrrole-2,5-dione**

Ce produit est obtenu selon le procédé de la préparation 3 en utilisant la N-(2- aminoéthyl)-N-[2-(diméthylamino) éthyl]amine à la place de la benzylamine.

**EXEMPLE 1 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexabydrofuro[3',4':6,7]naphtho[2,3-*d*][1,3]dioxol-5-yl]amino}-1-méthyl-1*H*-pyrrole-2,5-dione**

A une solution de 4 mmol du composé de la préparation 2 et de 3,6 mmol du composé de la préparation 1 dans 50 ml de diméthylformamide anhydre sous atmosphère inerte, sont ajoutées 4,4 mmol de triéthylamine. Le mélange réactionnel est agité à température ambiante pendant 24 heures. Le solvant est évaporé sous pression réduite et le résidu est repris dans un mélange d'eau (120 ml) et de dichlorométhane (200 ml). La phase aqueuse est extraite avec du dichlorométhane (3 x 40 ml). Les phases organiques sont séchées sur sulfate de magnésium, filtrées et évaporées sous pression réduite. Une chromatographie sur gel de silice (dichlorométhane/acétone : 95/5) permet d'isoler le produit attendu.
**Spectrométrie de masse (IC/$NH_3$) : m/z = 640 $[M+NH_4]^+$**

**EXEMPLE 2 :**

**3-{[(5S,5aS,8aR,9R)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-méthyl-1*H*-pyrrole-2,5-dione**

A une solution de 1,34 mmol du composé de l'exemple 1 dissous dans 125 ml de méthanol, sont ajoutées 8 g de résine Dowex (50 x 2 - 200) préalablement rincée avec de l'eau puis du méthanol. Le mélange réactionnel est agité pendant 18 heures, puis filtré et rincé à l'acétone. Les solvants sont évaporés sous pression réduite. Le produit attendu est obtenu après purification par chromatographie sur gel de silice (dichlorométhane/acétone : 9/1) et recristallisation

dans un mélange benzène/heptane.
**Point de fusion : 238-241°C**
**Spectrométrie de masse (IC/$NH_3$): m/z = 526 $[M+NH_4]^+$**

**EXEMPLE 3 :**

**3-{[(5S,5aS,8aR,9R)-9-(4-*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-benzyl-1*H*-pyrrole-2,5-dione**

Ce produit est obtenu selon le procédé de l'exemple 1 en utilisant le composé de la préparation 3 à la place du composé de la préparation 2.
**Spectrométrie de masse (IC/$NH_3$) : m/z = 715 $[M+NH_4]^+$**

**EXEMPLE 4 :**

**3-{[(5S,5aS,8aR,9R)-9-(4-hydroxy-3,5-diméthoxyphényl)-8-oxo-5,5a,6,8,8a,9-hexahydrofuro[3',4':6,7]naphtho[2,3-*d*][1,3]dioxol-5-yl]amino}-1-benzyl-1*H*-gyrrole-2,5-dione**

Ce produit est obtenu selon le procédé de l'exemple 2 en utilisant le composé de l'exemple 3.
**Point de fusion : 220-223°C**
**Spectrométrie de masse (IC/$NH_3$): m/z = 602 $[M+NH_4]^+$**

**EXEMPLE 5 :**

**3-1[(5*S*,5a*S*,8a*R*,9*R*)-9-(4-{[*tert*-Butyl(diméthyl)silyl]-oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-(4-fluorobenzyl)-1*H*-pyrrole-2,5-dione**

Ce produit est obtenu selon le procédé de l'exemple 1 en utilisant le composé de la préparation 4 à la place du composé de la préparation 2.
**Point de fusion : 182-184°C**
**Spectrométrie de masse (IC/$NH_3$) : m/z = 734 $[M+NH_4]^+$**

**EXEMPLE 6:**

**3-{[(5*S*,5a*S*,8a*R*,9*R*)-9-(4-Hydroxy-3,5-diméthoxyphényl)-8-oxo-5,5a,6,8,8a,9-hexahydrofuro[3',4':6,7]naphtho[2,3-*d*][1,3]dioxol-5-yl]-amino}-1-(4-fluorobenzyl)-1*H*-pyrrole-2,5-dione**

Ce produit est obtenu selon le procédé de l'exemple 2 en utilisant le composé de l'exemple 5.
**Point de fusion : 214-216°C**
**Spectrométrie de masse (IC/$NH_3$) : m/z = 620 $[M+NH_4]^+$**

**EXEMPLE 7 :**

**3-{[(5*S*,5a*S*,8a*R*,9*R*)-9-(4-{[*tert*-Butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxal-5-yl]amino}-1-[4-(trifluorométhyl)benzyl]-1*H*-pyrrole-2,5-dione**

Ce produit est obtenu selon le procédé de l'exemple 1 en utilisant le composé de la préparation 5 à la place du composé de la préparation 2.
**Point de fusion : 180-182°C**
**Spectrométrie de masse (IC/$NH_3$) : m/z = 784 $[M+NH_4]^+$**

**EXEMPLE 8 :**

**3-{[(5*S*,5a*S*,8a*R*,9*R*)-9-(4-Hydroxy-3,5-diméthoxyphényl)-8-oxo-5,5a,6,8,8a,9-hexahydrofuro[3',4':6,7]naphtho[2,3-*d*][1,3]dioxol-5-yl]-amino}-1-[4-(trifluorométhyl)henzyl]-1*H*-pyrrole-2,5-dione**

Ce produit est obtenu selon le procédé de l'exemple 2 en utilisant le composé de l'exemple 7.
**Point de fusion : 184-186°C**

**Spectrométrie de masse** (IC/$NH_3$) : m/z = 670 $[M+NH_4]^+$

**EXEMPLE 9 :**

***N*-{4-[(3-{[(5*S*,5a*S*,8a*R*,9*R*)-9-(4-{[*tert*-Butyl(diméthyl)silyl]oxy}-3,5-diméthoxyphényl)-8-oxo-5,5a,6,8,8a,9-hexahydrofuro-[3',4':6,7]naphtho[2,3-*d*][1,3]-dioxol-5-yl]amino}-2,5-dioxo-2,5-dihydro-1*H*-pyrrol-1-yl)méthyl]phényl}-acétamide**

Ce produit est obtenu selon le procédé de l'exemple 1 en utilisant le composé de la préparation 6 à la place du composé de la préparation 2.
**Point de fusion :** 177-179°C
**Spectrométrie de masse** (IC/$NH_3$) : m/z = 773 $[M+NH_4]^+$

**EXEMPLE 10 :**

***N*-{4-[(3-{[(5*S*,5a*S*,8a*R*,9*R*)-9-(4-hydroxy-3,5-diméthoxyphényl)-5-oxo-5,5a,6,8,8a,9-hexahydrofuro[3',4':6,7]naphtho[2,3-*d*][1,3]dioxol-5-yl]-amino}-2,5-dioxo-2,5-dihydro-1*H*-pyrrol-1-yl)méthyl]phényl}-acétamide**

Ce produit est obtenu selon le procédé de l'exemple 2 en utilisant le composé de l'exemple 9.
**Point de fusion :** 200-204°C (décomposition)
**Spectrométrie de masse** (IC/$NH_3$) : m/z = 659 $[M+NH_4]^+$

**EXEMPLE 11 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-[2-hydroxy-1-(hydroxyméthyl)éthyl]-1*H*-pyrrole-2,5-dione**

Ce produit est obtenu selon le procédé de l'exemple 1 en utilisant le composé de la préparation 7 à la place du composé de la préparation 2.

**EXEMPLE 12** :

**3-{[(5*S*,5a*S*,8a*R*,9*R*)-9-(4-hydroxy-3,5-diméthoxyphényl)-8-oxa-5,5a,6,8,8a,9-hexahydrofuro[3',4':6,7]naphtho[2,3-*d*][1,3]dioxol-5-yl]amino}-1-[2-hydroxy-1-(hydroxyméthyt)éthyl]-1*H*-pyrrole-2,5-dione**

Ce produit est obtenu selon le procédé de l'exemple 2 en utilisant le composé de l'exemple 11.

**EXEMPLE 13 :**

**Acide (3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl] amino}-2,5-dioxo-2,5-dihydro-1*H*-pyrrol-1-yl)acétique**

Ce produit est obtenu selon le procédé de l'exemple 1 en utilisant le composé de la préparation 8 à la place du composé de la préparation 2.

**EXEMPLE 14 :**

**Acide (3-{[(5*S*,5a*S*,8a*R*,9*R*)-9-(4-hydroxy-3,5-diméthaxyphényl)-8-oxa-5,5a,6,8,8a,9-hexahydrofuro[3',4':6,7]naphtho[2,3-*d*][1,3]dioxol-5-yl]amino}-2,5-dioxo-2,5-dihydro-1*H*-pyrrol-1-yl)acétique**

Ce produit est obtenu selon le procédé de l'exemple 2 en utilisant le composé de l'exemple 13.

**EXEMPLE 15 :**

**Acétate de (3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a, 9-hexahydrofuro[3',4':6,7]naphtho[2,3-*d*][1,3] dioxol-5-yl]amino}-2,5-dioxo-2,5-dihydro-1*H*-pyrrol-1-yl) méthyle**

Ce produit est obtenu selon le procédé de l'exemple 1 en utilisant le composé de la préparation 9 à la place du composé de la préparation 2.

**EXEMPLE 16 :**

**Acétate de (3-{[(5*S*,5a*S*,8a*R*,9*R*)-9-(4-hydroxy-3,5-diméthoxyphényl)-8-oxo-5,5a,6,8,8a,9-hexahydrofuro[3',4': 6,7]naphtho[2,3-*d*][1,3]dioxol-5-yl]amino}-2,5-dioxo-2,5-dihydro-1*H*-pyrrol-1-yl) méthyle**

Ce produit est obtenu selon le procédé de l'exemple 2 en utilisant le composé de l'exemple 15.

**EXEMPLE 17 :**

**Acide 6-(3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a, 9-hexahydrofuro[3',4':6,7]naphtho[2,3-*d*][1,3]dioxol-5-yl] amino}-2,5-dioxo-2,5-dihydro-1*H*-pyrrol-1-yl)hexanoïque**

Ce produit est obtenu selon le procédé de l'exemple 1 en utilisant le composé de la préparation 10 à la place du composé de la préparation 2.

**EXEMPLE 18 :**

**Acide 6-(3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7] naphtho[2,3-*d*][1,3]dioxol-5-yl]amino}-2,5-dioxo-2,5-dihydro-1*H*-pyrrol-1-yl)hexanoïque**

Ce produit est obtenu selon le procédé de l'exemple 2 en utilisant le composé de l'exemple 17.
**Point de fusion : 150-152°C**
**Spectrométrie de masse (IC/$NH_3$) : m/z = 626 $[M+NH_4]^+$**

**EXEMPLE 19 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-butyl-1*H*-pyrrole-2,5-dione**

Ce produit est obtenu selon le procédé de l'exemple 1 en utilisant le composé de la préparation 11 à la place du composé de la préparation 2.

**EXEMPLE 20 :**

**3-{[(5*S*,5a*S*,8a*R*,9*R*)-9-(4-hydraxy-3,5-diméthaxyphényl)-8-oxo-5,5a,6,8,8a,9-hexahydrofuro[3',4':6,7]naphtho [2,3-*d*][1,3]dioxol-5-yl]amina}-1-butyl-1*H*-pyrrale-2,5-dione**

Ce produit est obtenu selon le procédé de l'exemple 2 en utilisant le composé de l'exemple 19.
**Spectrométrie de masse (IC/$NH_3$) : m/z = 568 $[M+NH_4]^+$**

**EXEMPLE 21 :**

**3-{[(5*S*,5a*S*,8a*R*,9*R*)-8-oxo-9-(3,4,5-triméthobyphényl)-5,5a,6,8,8a,9-hexahydrofuro [3',4':6,7]naphtho[2,3-*d*] [1,3]dioxol-5-yl]amino}-1-méthyl-1*H*-pyrrole-2,5-dione**

Ce produit est obtenu selon le procédé de l'exemple 1 en utilisant le composé de la préparation 12 à la place du composé de la préparation 1.

**EXEMPLE 22 :**

**3-{[(5*S*,5a*S*,8a*R*,9*R*)-9-(4-([*tert*-butyl(diméthyl)silyl]oxy}-3,5-diméthoxyphényl)-8-oxo-5,5a,6,8,8a,9-hexahydrofuro[3',4';6,7]naphtho[2,3-*d*][1,3]dioxol-5-yl]amino}-1,4-diméthyl-1*H*-pyrrole-2,5-dione**

Ce produit est obtenu selon le procédé de l'exemple 1 en utilisant le composé de la préparation 13 à la place du composé de la préparation 2.

**EXEMPLE 23 :**

**3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho[2,3-*d*][1,3]dioxol-5-yl]amino}-1,4-diméthyl-1*H*-pyrrole-2,5-dione**

Ce produit est obtenu selon le procédé de l'exemple 2 en utilisant le composé de l'exemple 22.
**Point de fusion :** **172-176°C**
**Spectrométrie de masse** **(IC/$NH_3$) : m/z = 540 [M+$NH_4$]$^+$**

**EXEMPLE 24 :**

**3-[[(5*S*,5a*S*,8a*R*,9*R*)-9-(4-{[*tert*-butyl(diméthyl)silyl]oxy}-3,5-dimêthoxyphényl)-8-oxo-5,5a,6,8,8a,9-hexahydrofuro[3',4':6,7]naphtho[2,3-*d*][1,3]dioxol-5-yl](méthyl)amino]-1-méthyl-1*H*-pyrrole-2,5-dione**

Ce produit est obtenu selon le procédé de l'exemple 1 en utilisant le composé de la préparation 14 à la place du composé de la préparation 1.

**EXEMPLE 25 :**

**3-[[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho[2,3-*d*][1,3]dioxol-5-yl](méthyl)amino]-1-méthyl-1*H*-pyrrole-2,5-dione**

Ce produit est obtenu selon le procédé de l'exemple 2 en utilisant le composé de l'exemple 24.

**EXEMPLE 26 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-(2-hydroxyéthyl)-1*H*-pyrrole-2,5-dione**

Ce produit est obtenu selon le procédé de l'exemple 1 en utilisant le composé de la préparation 15 à la place du composé de la préparation 2.

**EXEMPLE 27 :**

**3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho[2,3-*d*][1,3]dioxol-5-yl]amino}-1-(2-hydroxyéthyl)-1*H*-pyrrole-2,5-dione**

Ce produit est obtenu selon le procédé de l'exemple 2 en utilisant le composé de l'exemple 26.

**EXEMPLE 28 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-(2-méthoxyéthyl)-1*H*-pyrrole-2,5-dione**

Ce produit est obtenu selon le procédé de l'exemple 1 en utilisant le composé de la préparation 16 à la place du composé de la préparation 2.

**EXEMPLE 29 :**

**3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-(2-méthoxyéthyl)-1*H*-pyrrole-2,5-dione**

Ce produit est obtenu selon le procédé de l'exemple 2 en utilisant le composé de l'exemple 28.

**EXEMPLE 30 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-allyl-1*H*-pyrrole-2,5-dione**

Ce produit est obtenu selon le procédé de l'exemple 1 en utilisant le composé de la préparation 17 à la place du composé de la préparation 2.

**EXEMPLE 31 :**

**3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naghtho [2,3-*d*][1,3]dioxol-5-yl]amino}-1-allyl-1*H*-pyrrole-2,5-dione**

Ce produit est obtenu selon le procédé de l'exemple 2 en utilisant le composé de l'exemple 30.
**Point de fusion : 180-183°C**
**Spectrométrie de masse (IC/$NH_3$) : m/z = 552 [M+$NH_4$]$^+$**

**EXEMPLE 32 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-[2-(1-pipéridinyl)éthyl]-1*H*-pyrrole-2,5-dione**

Ce produit est obtenu selon le procédé de l'exemple 1 en utilisant le composé de la préparation 18 à la place du composé de la préparation 2.

**EXEMPLE 33 :**

**3-{[(5*S*,5a*S*,8a*R*,9*R*)-9-(4-hydroxy-3,5-dimethoxyphényl)-8-oxo-5,5a,6,8,8a,9-hexahydrofuro[3',4':6,7]naphtho [2,3-*d*][1,3]dioxol-5-yl]amino}-1-[2-(1-pipéridinyl) éthyl]-1*H*-pyrrole-2,5-dione**

Ce produit est obtenu selon le procédé de l'exemple 2 en utilisant le composé de l'exemple 32.

**EXEMPLE 34 :**

**3-{[(5*S*,5a*S*,8a*R*,9*R*)-9-(4-{[*tert*-butyl(diméthyl)sily]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-[2-(4-morpholinyl)éthyl]-1*H*-pyrrole-2,5-dione**

Ce produit est obtenu selon le procédé de l'exemple 1 en utilisant le composé de la préparation 19 à la place du composé de la préparation 2.

**EXEMPLE 35 :**

**3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-[2-(4-morpholinyl)éthyl]-1*H*-pyrrole-2,5-dione**

Ce produit est obtenu selon le procédé de l'exemple 2 en utilisant le composé de l'exemple 34.

**EXEMPLE 36 :**

**4-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1,2-dihydro-3,6-pyridazinedione**

Ce produit est obtenu selon le procédé de l'exemple 1 en utilisant le composé de la préparation 20 à la place du composé de la préparation 2.

**EXEMPLE 37 :**

**4-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho[2,3-*d*][1,3]dioxol-5-yl]amino}-1,2-dihydro-3,6-**pyridazinedione

Ce produit est obtenu selon le procédé de l'exemple 2 en utilisant le composé de l'exemple 36.

**EXEMPLE 38 :**

**Acétate de 2-(3-{[(5*S*,5a*S*,8a*R*,9*R*)-9-(4-([*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3] dioxol-5-yl]amino}-2,5-dioxo-2,5-dihydro-1*H*-pyrrole-1-yl)éthyle**

Ce produit est obtenu selon le procédé de l'exemple 1 en utilisant le composé de la préparation 21 à la place du composé de la préparation 2.

**EXEMPLE 39 :**

**Acétate de 2-(3-{[(5*S*,5a*S*,8a*R*,9*R*)-9-(4-hydroxy-3,5-diméthoxyphényl)-8-oxo-5,5a,6,8, 8a,9-hexahydrofuro[3',4':6,7]naphtho[2,3-*d*][1,3]dioxol-5-yl]amino}-2,5-dioxo-2,5-dibydro-1*H*-pyrrole-1-yl)éthyle**

Ce produit est obtenu selon le procédé de l'exemple 2 en utilisant le composé de l'exemple 38.
**Spectrométrie de masse (IC/$NH_3$) : m/z = 598 $[M+NH_4]^+$**

**EXEMPLE 40 :**

**Acide 7-(3-{[(5*S*,5a*S*,8a*R*,9*R*)-9-(4-{[*tert*-butyl(diméthyl)silyl]oxyl-3,5-diméthoxyphényl)-8-oxo-5,5a,6,8,8a,9-hexahydrofuro[3',4':6,7]naphtho[2,3-*d*][1,3] dioxol-5-yl]amino}-2,5-dioxo-2,5-dihydro-1*H*-pyrrole-1-yl)heptanoïque**

Ce produit est obtenu selon le procédé de l'exemple 1 en utilisant le composé de la préparation 22 à la place du composé de la préparation 2.

**EXEMPLE 41 :**

**Acide 7-(3-{[(5*S*,5a*S*,8a*R*,9*R*)-9-(4hydroxy-3,5-diméthoxyphényl)-8-oxo-5,5a,6,8,8a,9-hexahydrofuro[3',4':6,7] naphiho[2,3-*d*][1,3]dioxol-5-yl]amino}-2,5-dioxo-2,5-dihydro-1*H*-pyrrole-1-yl)heptanoïque**

Ce produit est obtenu selon le procédé de l'exemple 2 en utilisant le composé de l'exemple 40.

**EXEMPLE 42 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-(2,3-dihydroxypropyl)-1*H*-pyrrole-2,5-dione**

Ce produit est obtenu selon le procédé de l'exemple 1 en utilisant le composé de la préparation 23 à la place du composé de la préparation 2.

**EXEMPLE 43 :**

**3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-(2,3-dihydroxypropyl)-1*H*-pyrrole-2,5-dione**

Ce produit est obtenu selon le procédé de l'exemple 2 en utilisant le composé de l'exemple 42.

**EXEMPLE 44 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-[2-(4-méthyl-1-pipérazinyl)éthyl]-1*H*-pyrrole-2,5-dione**

Ce produit est obtenu selon le procédé de l'exemple 1 en utilisant le composé de la préparation 24 à la place du composé de la préparation 2.

**EXEMPLE 45 :**

**3-{[(5*S*,5a*S*-8a*R*-9*R*)-9-(4-hydroxy-3,5-diméthoxyphényl)-8-oxo-5,5a,6,8,8a,9-hexahydrafura[3',4':6,7]naphtha [2,3-*d*][1,3]dioxol-5-yl]amino}-1-[2-(4-méthyl-1-pipérazinyl)éthyl]-1*H*-pyrrole-2,5-dione**

Ce produit est obtenu selon le procédé de l'exemple 2 en utilisant le composé de l'exemple 44.

**EXEMPLE 46 :**

**3-{[(5*S*,5a*S*,8a*R*,9*R*)-9-(4-{[*tert*-butyl(diméthyl)silyl]oxy}-3,5-diunéthoxyphényl)-8-oxo-5,5a,6,8,8a,9-hexahydrofuro[3',4':6,7]naphtho[2,3-*d*][1,3]dioxol-5-yl]amino}-1-[2-(diméthylamino)éthyl]-1*H*-pyrrole-2,5-dione**

Ce produit est obtenu selon le procédé de l'exemple 1 en utilisant le composé de la préparation 25 à la place du composé de la préparation 2.

**EXEMPLE 47 :**

**3-{[(5*S*,5a*S*,8a*R*,9*R*)-9-(4-hydroxy-3,5-diméthoxyphényl)-8-oxo-5,5a,6,8,8a,9-hexahydrafuro[3',4':6,7]naphtho [2,3-*d*][1,3]dioxol-5-yl]amino}-1-[2-(diméthylamino) éthyl]-1*H*-pyrrole-2,5-dione**

Ce produit est obtenu selon le procédé de l'exemple 2 en utilisant le composé de l'exemple 46.

**EXEMPLE 48 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-[2-(butylamina)éthyl]-1*H*-pyrrole-2,5-dione**

Ce produit est obtenu selon le procédé de l'exemple 1 en utilisant le composé de la préparation 26 à la place du composé de la préparation 2.

**EXEMPLE 49 :**

**3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho [2,3-*d*][1,3]dioxol-5-yl]amino}-1-[2-(butylamino) éthyl]-1*H*-pyrrole-2,5-dione**

Ce produit est obtenu selon le procédé de l'exemple 2 en utilisant le composé de l'exemple 48.

**EXEMPLE 50 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-(2-{[2-diméthylamino)éthyl]amino}éthyl)-1*H*-pyrrole-2,5-dione**

Ce produit est obtenu selon le procédé de l'exemple 1 en utilisant le composé de la préparation 27 à la place du composé de la préparation 2.

**EXEMPLE 51 :**

**3-{[(5*S*,5a*S*,8a*R*,9*R*)-9-(4-hydroxy-3,5-diméthoxyphényl)-8-oxo-5,5a,6,8,8a,9-hexahydrofuro[2,3-*d*][1,3]dioxol-5-yl]amino}-1-(2-{[2-(diméthylamino)éthyl] amino}éthyl]-1*H*-pyrrole-2,5-dione**

Ce produit est obtenu selon le procédé de l'exemple 2 en utilisant le composé de l'exemple 50.

**EXEMPLE 52 :**

**3-{[(5*S*,5a*S*,8a*R*,9*R*)-9-(4-{[*tert*-butyl(diméthyl)silyl]oxy}-3,5-diméthoxyphényl)-8-oxo-5,5a,6,8,8a,9-hexahydrofuro[3',4':6,7]naphtho[2,3-*d*][1,3]dioxol-5-yl]amino}-1-[2-(4-méthyl-1-pipérazinyl)-2-oxoéthyl]-1*H*-pyrrole-2,5-dione**

Ce produit est obtenu en faisant réagir la 1-méthylpipérazine avec le composé de l'exemple 13.

**EXEMPLE 53 :**

**3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7]naphtho[2,3-*d*][1,3]dioxol-5-yl]amino}-1-[2-(4-méthyl-1-pipérazinyl)-2-oxoéthyl]-1*H*-pyrrole-2,5-dione**

Ce produit est obtenu selon le procédé de l'exemple 2 en utilisant le composé de l'exemple 52.

**ETUDE PHARMACOLOGIQUE DES COMPOSES DE L'INVENTION**

**EXEMPLE 54 : Activité in vitro**

Six lignées cellulaires ont été utilisées :

- ➢ 1 leucémie murine : L1210
- ➢ 1 carcinome épidermoïde humain : KB-3-1
- ➢ 1 carcinome pulmonaire humain, non à petites cellules : A549
- ➢ 1 mélanome : A375
- ➢ 1 neuroblastome : SK-N-MC
- ➢ 1 fibrosarcome : HT1080

Les cellules sont cultivées dans du milieu de culture RPMI 1640 complet contenant 10 % de sérum de veau foetal, 2 mM de glutamine, 50 U/ml de pénicilline, 50 μg/ml de streptomycine et 10 mM d'Hepes, pH : 7,4. Les cellules sont réparties dans des microplaques et exposées aux composés cytotoxiques pendant 4 temps de doublement, soit 48 heures. Le nombre de cellules viables est ensuite quantifié par un essai colorimétrique, le Microculture Tetrazolium Assay (J. Carmichael et al., Cancer Res. ; 47, 936-942, (1987)). Les résultats sont exprimés en $IC_{50}$, concentration en cytotoxique qui inhibe à 50 % la prolifération des cellules traitées.

Lors de ce test, les composés des exemples 2 et 4 présentent une $IC_{50}$ sur L1210 de 54 nM et 29 nM respectivement.

| *Lignées cellulaires* | *IC50 (nM) Exemple 2* |
|---|---|
| *L1210* | *54* |
| *KB-3-1* | *66, 6* |

(suite)

| *Lignées cellulaires* | *IC50 (nM) Exemple 2* |
|---|---|
| *A549* | *110* |
| *A375* | *44,2* |
| *SK-N-MC* | *106* |
| *HT1080* | *75,8* |

**EXEMPLE 55 : Action sur le cycle cellulaire**

Les cellules L1210 sont incubées pendant 21 heures à 37°C en présence de différentes concentrations en produits testés. Les cellules sont ensuite fixées par de l'éthanol à 70 % (v/v), lavées deux fois dans du PBS et incubées 30 minutes à 20°C dans du PBS contenant 100 μg/ml de RNAse et 50 μg/ml d'iodure de propidium. Les résultats sont exprimés en pourcentage des cellules accumulées en phase G2+M après 21 heures par rapport au témoin (témoin : 20 %). Les composés des exemples 2 et 4 induisent une accumulation à 70 % et 81 % respectivement des cellules en phase G2+M après 21 heures à une concentration de 250 nM.

**EXEMPLE 56 : Activité in vivo**

** Activité antitumorale des composés sur la leucémie P388 et sur le carcinome pulmonaire humain A549 :*

La lignée P388 (leucémie murine) a été fournie par le National Cancer Institute (Frederick, USA). Les cellules tumorales ($10^6$ cellules) ont été inoculées au jour 0 dans la cavité péritonéale de souris BDF1 femelles (Iffa-Credo, France) pesant de 18 à 20 g (groupes de 6 animaux). Les produits ont été administrés au jour 1 par voie intraveineuse. L'activité antitumorale est exprimée en % de T/C :

$$\% \text{ T/C} = \frac{\text{Temps de survie médian des animaux traités}}{\text{Temps de survie médian des animaux contrôles}} \times 100$$

A titre indicatif, le composé de l'exemple 2 est actif sur la leucémie P388 et sur le carcinome pulmonaire humain A549 et il induit un T/C de > 575 % à 50 mg/kg, avec 50 % des animaux survivants au jour 60 et un T/C de 225 % à 3,12 mg/kg pour P388 et A549 respectivement.

**Revendications**

1. Composés de formule (I) :

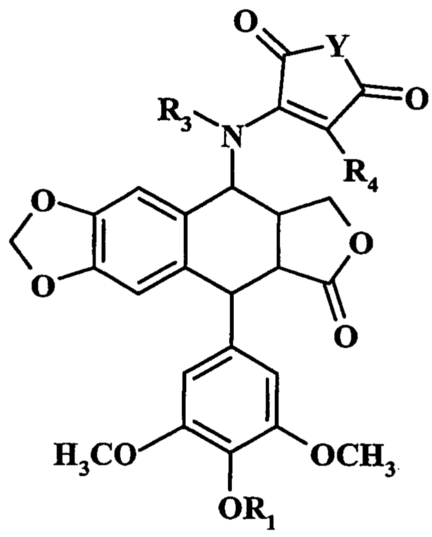

(I)

dans laquelle :

$R_1$ représente un groupement choisi parmi hydrogène, alkyle ($C_1$-$C_6$) linéaire ou ramifié, aryle, arylalkyle ($C_1$-$C_6$) linéaire ou ramifié, hétéroaryle, hétéroarylalkyle ($C_1$-$C_6$) linéaire ou ramifié, alkylcarbonyle ($C_1$-$C_6$) linéaire ou ramifié, arylcarbonyle, arylalkylcarbonyle ($C_1$-$C_6$) linéaire ou ramifié, alkoxycarbonyle ($C_1$-$C_6$) linéaire ou ramifié, aryloxycarbonyle, arylalkoxycarbonyle ($C_1$-$C_6$) linéaire ou ramifié, hétérocycloalkoxycarbonyle, alkylsulfonyle ($C_1$-$C_6$) linéaire ou ramifié, arylsulfonyle, arylalkylsulfonyle ($C_1$-$C_6$) linéaire ou ramifié, phosphonique, ou Si $(R_a)_2R_b$ dans laquelle $R_a$, $R_b$, identiques ou différents, représentent chacun un groupement choisi parmi alkyle ($C_1$-$C_6$) linéaire ou ramifié ou aryle,
Y représente un groupement choisi parmi HN-NH ou N-$R_2$ dans laquelle :

$R_2$ représente un groupement choisi parmi hydrogène, alkyle ($C_1$-$C_6$) linéaire ou ramifié, aryle, hétéroaryle, cycloalkyle, hétérocycloalkyle, alkényle ($C_2$-$C_6$) linéaire ou ramifié, alkynyle ($C_2$-$C_6$) linéaire ou ramifié ou un groupement de formule -$T_1$-$R_5$ dans laquelle :

- ♦ $T_1$ représente un groupement choisi parmi une chaîne alkylène ($C_1$-$C_6$) linéaire ou ramifiée éventuellement substituée par un ou plusieurs groupements choisi parmi hydroxy ou alkoxy ($C_1$-$C_6$) linéaire ou ramifié, une chaîne alkénylène ($C_2$-$C_6$) linéaire ou ramifiée ou une chaîne alkynylène ($C_2$-$C_6$) linéaire ou ramifiée,
- ♦ $R_5$ représente un groupement choisi parmi hydroxy, alkoxy ($C_1$-$C_6$) linéaire ou ramifié, alkylcarbonyle ($C_1$-$C_6$) linéaire ou ramifié, alkylcarbonyloxy ($C_1$-$C_6$) linéaire ou ramifié, alkoxycarbonyle ($C_1$-$C_6$) linéaire ou ramifié, carboxy, halogène, trihalogénométhyle, aryle, hétéroaryle, cycloalkyle, hétérocycloalkyle, $NR_cR_d$ dans lequel $R_c$, $R_d$, identiques ou différents, représentent chacun un groupement choisi parmi hydrogène, alkyle ($C_1$-$C_6$) linéaire ou ramifié, aminoalkyle ($C_1$-$C_6$) linéaire ou ramifié dans lequel la partie amino est éventuellement substituée par un ou deux groupements identiques ou différents, alkyle ($C_1$-$C_6$) linéaire ou ramifié, hydroxyalkyle ($C_1$-$C_6$) linéaire ou ramifié, alkoxy ($C_1$-$C_6$) alkyle ($C_1$-$C_6$) linéaire ou ramifié
  ou $C(O)NR'_cR'_d$ dans lequel $R'_c$, $R'_d$, identiques ou différents, représentent chacun un groupement choisi parmi hydrogène, alkyle ($C_1$-$C_6$) linéaire ou ramifié, aminoalkyle ($C_1$-$C_6$) linéaire ou ramifié dans lequel la partie amino est éventuellement substituée par un ou deux groupements identiques ou différents, alkyle ($C_1$-$C_6$) linéaire ou ramifié, hydroxyalkyle ($C_1$-$C_6$) linéaire ou ramifié, alkoxy ($C_1$-$C_6$) alkyle ($C_1$-$C_6$) linéaire ou ramifié ou $R'_c$ et $R'_d$ forment ensemble avec l'atome d'azote qui les porte, un hétérocycloalkyle,

$R_3$ représente un groupement choisi parmi hydrogène, alkyle ($C_1$-$C_6$) linéaire ou ramifié, cycloalkyle, cycloalkylalkyle ($C_1$-$C_6$) linéaire ou ramifié, aryle ou arylalkyle ($C_1$-$C_6$) linéaire ou ramifié,
$R_4$ représente un groupement choisi parmi hydrogène, alkyle ($C_1$-$C_6$) linéaire ou ramifié,

leurs énantiomères, diastéréoisomères ainsi que leurs sels d'addition à un acide ou à une base pharmaceutiquement acceptable,
étant entendu que :

* *par aryle,* on comprend un groupement choisi parmi phényle, biphényle, naphtyle, dihydronaphtyle, tétrahydronaphtyle, indényle, indanyle, et benzocyclobutyle, chacun de ces groupements comportant éventuellement une ou plusieurs substitutions, identiques ou différentes, choisies parmi halogène, hydroxy, alkyle ($C_1$-$C_6$) linéaire ou ramifié, alkoxy ($C_1$-$C_6$) linéaire ou ramifié, cyano, nitro, amino, alkylamino ($C_1$-$C_6$) linéaire ou ramifié, dialkylamino ($C_1$-$C_6$) linéaire ou ramifié, carboxy, alkoxycarbonyle ($C_1$-$C_6$) linéaire ou ramifié, trihalogénoalkyle ($C_1$-$C_6$) linéaire ou ramifié, alkylcarbonyloxy ($C_1$-$C_6$) linéaire ou ramifié, alkylcarbonyle ($C_1$-$C_6$) linéaire ou ramifié, et aminocarbonyle dans laquelle la partie amino est éventuellement substituée par un ou deux groupements, identiques ou différents, alkyle ($C_1$-$C_6$) linéaire ou ramifié,
* *par hétéroaryle,* on comprend un groupement mono- ou bicyclique aromatique ou un groupement bicyclique dont l'un des cycles est aromatique et l'autre cycle est partiellement hydrogéné, de 5 à 12 chaînons, comportant au sein du système cyclique de un à trois hétéroatomes, identiques ou différents, choisis parmi oxygène, azote et soufre, ledit groupement hétéroaryle pouvant éventuellement être substitué par un ou plusieurs groupements identiques ou différents, choisis parmi les substituants définis précédemment dans le cas du groupement aryle ; parmi les groupements hétéroaryles, on peut citer à titre non limitatif le groupement pyridyle, pyrrolyle, thiényle, furyle, pyrazinyle, isothiazolyle, thiazolyle, oxazolyle, isoxazolyle, pyrimidinyle, indolyle, benzofuranyle, benzothiényle, quinolyle, isoquinolyle, benzo[1,4]dioxynyle, 2,3-dihydrobenzo[1,4]dioxynyle,
* *par cycloalkyle,* on comprend un groupement mono- ou bicyclique, saturé ou insaturé mais sans caractère aromatique, comportant de 3 à 12 atomes de carbone, étant éventuellement substitué par un ou plusieurs groupements, identiques ou différents, choisis parmi halogène, alkyle ($C_1$-$C_6$) linéaire ou ramifié, trihalogénoalkyle ($C_1$-$C_6$) linéaire ou ramifié, hydroxy, amino, alkylamino ($C_1$-$C_6$) linéaire ou ramifié et dialkylamino ($C_1$-$C_6$) linéaire ou ramifié ; parmi les groupements cycloalkyles, on peut citer à titre non limitatif, le groupement cyclopropyle, cyclobutyle, cyclopentyle, cyclohexyle,
* *par hétérocycloalkyle,* on comprend un cycloalkyle tel que défini précédemment, comportant au sein du système cyclique, de un à deux hétéroatomes, identiques ou différents, choisis parmi oxygène et azote, ledit hétérocycloalkyle étant éventuellement substitué par un ou plusieurs groupements identiques ou différents définis précédemment dans le cas du groupement cycloalkyle ; parmi les groupements hétérocycloalkyles, on peut citer à titre non limitatif le groupement pipéridyle, pipérazinyle, morpholyle.

**2.** Composés de formule (I) selon la revendication 1 **caractérisés en ce que** $R_1$ représente un atome d'hydrogène, leurs énantiomères, diastéréoisomères ainsi que leurs sels d'addition à un acide ou à une base pharmaceutiquement acceptables.

**3.** Composés de formule (I) selon l'une quelconque des revendications 1 à 2 **caractérisés en ce que** $R_3$ représente un atome d'hydrogène, leurs énantiomères, diastéréoisomères ainsi que leurs sels d'addition à un acide ou à une base pharmaceutiquement acceptables.

**4.** Composés de formule (I) selon l'une quelconque des revendications 1 à 3 **caractérisés en ce que** $R_4$ représente un atome d'hydrogène, ou un groupement méthyle, leurs énantiomères, diastéréoisomères ainsi que leurs sels d'addition à un acide ou à une base pharmaceutiquement acceptables.

**5.** Composés de formule (I) selon l'une quelconque des revendications 1 à 4 **caractérisés en ce que** Y représente un groupement HN-NH ou N-$R_2$ dans lequel $R_2$ représente un groupement alkyle ($C_1$-$C_6$) linéaire ou ramifié, alkényle ($C_2$-$C_6$) linéaire ou ramifié, ou un groupement de formule -$T_1$-$R_5$ dans laquelle $T_1$ et $R_5$ sont tels que définis dans la formule (I), leurs énantiomères, diastéréoisomères ainsi que leurs sels d'addition à un acide ou à une base pharmaceutiquement acceptables.

**6.** Composés de formule (I) selon l'une quelconque des revendications 1 à 5 **caractérisés en ce que** Y représente un groupement de formule $NR_2$ dans lequel $R_2$ représente un groupement méthyle, leurs énantiomères, diastéréoisomères ainsi que leurs sels d'addition à un acide ou à une base pharmaceutiquement acceptables.

**7.** Composés de formule (I) selon l'une quelconque des revendications 1 à 6 **caractérisés en ce que** Y représente un groupement de formule $NR_2$ dans lequel $R_2$ représente un groupement $T_1$-$R_5$ dans lequel $T_1$ représente une chaîne alkylène ($C_1$-$C_6$) linéaire ou ramifiée, et $R_5$ représente un groupement choisi parmi aryle, carboxy et alkylcarbonyloxy ($C_1$-$C_6$) linéaire ou ramifié, leurs énantiomères, diastéréoisomères ainsi que leurs sels d'addition à un

acide ou à une base pharmaceutiquement acceptables.

8. Composés de formule (I) selon l'une quelconque des revendications 1 à 7 **caractérisés en ce que** Y représente un groupement de formule $NR_2$ dans lequel $R_2$ représente un groupement $T_1$-$R_5$ dans lequel $T_1$ représente un groupement méthylène $-CH_2-$ et $R_5$ représente un groupement aryle, leurs énantiomères, diastéréoisomères ainsi que leurs sels d'addition à un acide ou à une base pharmaceutiquement acceptables.

9. Composés de formule (I) selon la revendication 1 qui sont le :

♦ 3{[(5*S*,5a*S*,8a*R*,9*R*)-9-(4-hydroxy-3,5-diméthoxyphényl)-8-oxo-5,5a,6,8,8a,9-hexahydrofuro[3',4':6,7] naphtho[2,3-*d*][1,3]dioxol-5-yl]amino}-1-méthyl-1*H*-pyrrole-2,5-dione ;
♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7] naphtho[2,3-*d*][1,3]dioxol-5-yl)amino}-1-benzyl-1*H*-pyrrole-2,5-dione ;
♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7] naphtho[2,3-*d*][1,3]dioxol-5-yl]amino}-1-(4-fluorobenzyl)-1*H*-pyrrole-2,5-dione ;
♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7] naphtho[2,3-*d*][1,3]dioxol-5-yl]amino}-1-[4-(trifluorométhyl)benzyl]-1*H*-pyrrole-2,5-dione ;
♦ *N*-{4-[(3-{[(5*S*,5a*S*,5a*S*,8a*R*,9*R*)-9-(4-hydroxy-3,5-diméthoxyphényl)-8-oxo-5,5a,6,8,8a,9-hexahydrofuro[3', 4':6,7]naphtho[2,3-*d*][1,3]dioxol-5-yl]amino}-2,5-dioxo-2,5-dihydro-1*H*-pyrrol-1-yl)méthyl]phényl}acétamide.
♦ Acide 6-(3-{[(5*S*,5a*S*,8a*R*,9*R*)-9-(4-hydroxy-3,5-diméthoxyphényl)-8-oxo-5,5a,6,8,8a,9-hexahydrofuro[3',4': 6,7]naphtho[2,3-*d*][1,3]dioxol-5-yl]amino}-2,5-dioxo-2,5-dihydro-1*H*-pyrrol-1-yl)hexanoïque
♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7] naphtho[2,3-*d*][1,3]dioxol-5-yl]amino}-1-butyl-1*H*-pyrrole-2,5-dione
♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7] naphtho[2,3-*d*][1,3]dioxol-5-yl]amino}-1-allyl-1*H*-pyrrole-2,5-dione
♦ Acétate de 2-(3-{[(5*S*,5a*S*,8a*R*,9*R*)-9-(4-hydroxy-3,5-diméthoxyphényl)-8-oxo-5,5a,6,8, 8a,9-hexahydrofuro [3',4':6,7]naphtho[2,3-*d*][1,3]dioxol-5-yl]amino}-2,5-dioxo-2,5-dihydro-1*H*-pyrrole-1-yl)éthyle
♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7] naphtho[2,3-*d*][1,3]dioxol-5-yl]amino}-1-(2,3-dihydroxypropyl)-1*H*-pyrrole-2,5-dione
♦ 3-{[(5*S*,5a*S*,8a*R*,9*R*)-9-(4-hydroxy-3,5-diméthoxyphényl)-8-oxo-5,5a,6,8,8a,9-hexahydrofuro[3',4':6,7] naphtho[2,3-*d*][1,3]dioxol-5-yl]amino}-1-[2-(diméthylamino) éthyl]-1*H*-pyrrole-2,5-dione

10. Procédé de préparation des composés de formule (I), **caractérisé en ce qu'**on utilise comme produit de départ un composé de formule (II) :

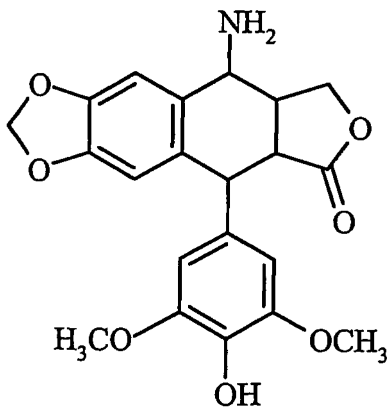

(II)

qui est soumis, en condition basique :

➢ <u>soit</u> à l'action d'un composé de formule (HT) :

$R'_1$ - X (III)

dans laquelle $R'_1$ représente un groupement choisi parmi alkyle ($C_1$-$C_6$) linéaire ou ramifié, aryle, arylalkyle

($C_1$-$C_6$) linéaire ou ramifié, hétéroaryle, hétéroarylalkyle ($C_1$-$C_6$) linéaire ou ramifié, alkylcarbonyle ($C_1$-$C_6$) linéaire ou ramifié, arylcarbonyle, arylalkylcarbonyle ($C_1$-$C_6$) linéaire ou ramifié, alkoxycarbonyle ($C_1$-$C_6$) linéaire ou ramifié, aryloxycarbonyle, arylalkoxycarbonyle ($C_1$-$C_6$) linéaire ou ramifié, hétérocycloalkoxycarbonyle, alkylsulfonyle ($C_1$-$C_6$) linéaire ou ramifié, arylsulfonyle, arylalkylsulfonyle ($C_1$-$C_6$) linéaire ou ramifié, phosphonique, ou $Si(R_a)_2R_b$ dans laquelle $R_a$, $R_b$, identiques ou différents, représentent chacun un groupement choisi parmi alkyle ($C_1$-$C_6$) linéaire ou ramifié ou aryle,
et X représente un atome d'hydrogène, un atome d'halogène ou un groupe partant usuel de la chimie organique, pour conduire aux composés de formule (IV/a):

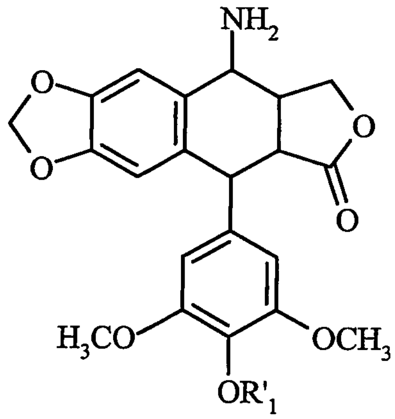

(IV/a)

dans laquelle $R'_1$ est tel que défini précédemment,
➢ <u>soit</u> à l'action d'un composé de formule (V) :

G-L (V)

dans laquelle G représente un groupement protecteur classique des fonctions hydroxy et L un groupe partant usuel de la chimie organique, pour conduire aux composés de formule (IV/b):

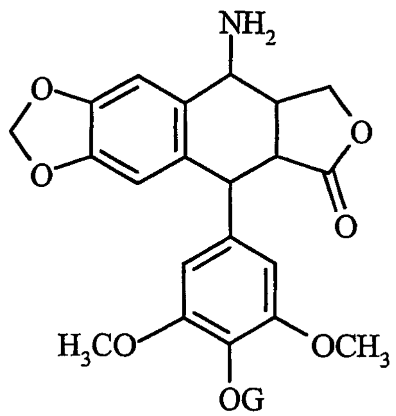

(IV/b)

dans laquelle G est tel que défini précédemment,
l'ensemble des composés de formule (IV/a) et (IV/b) formant les composés de formule (IV) :

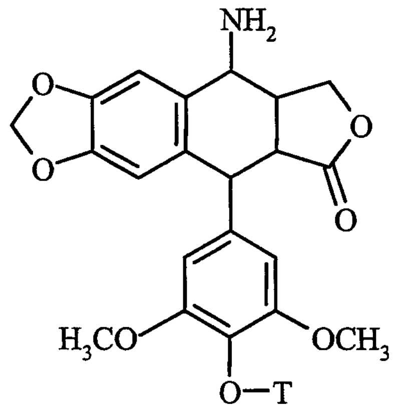

(IV)

dans laquelle T représente un groupement $R'_1$ ou G tels que définis précédemment,
composé de formule (IV), qui est soumis, en condition basique, à l'action d'un composé de formule (VI) :

$R'_3$-X' (VI)

dans laquelle $R'_3$ représente un groupement choisi parmi alkyle ($C_1$-$C_6$) linéaire ou ramifié, cycloalkyle, cycloalkylalkyle ($C_1$-$C_6$) linéaire ou ramifié, aryle ou arylalkyle ($C_1$-$C_6$) linéaire ou ramifié,
et X' représente un atome d'hydrogène, un atome d'halogène ou un groupe partant usuel de la chimie organique, pour conduire aux composés de formule (VII) :

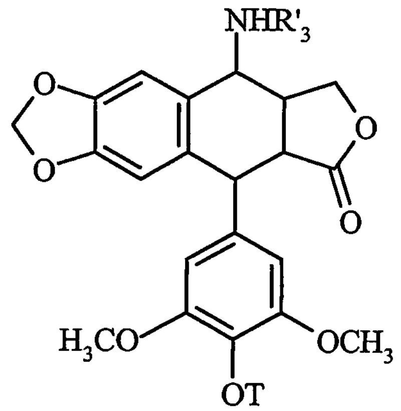

(VII)

dans laquelle $R'_3$ et T sont tels que définis précédemment,
l'ensemble des composés de formules (IV) et (VII) formant les composés de formule (VIII) :

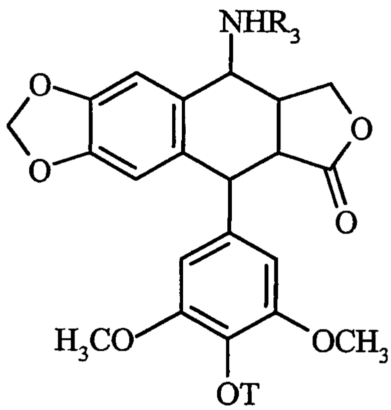

(VIII)

dans laquelle $R_3$ et T sont tels que définis dans la formule (I), composé de formule (VIII) que l'on traite en milieu basique par un composé de formule (IX):

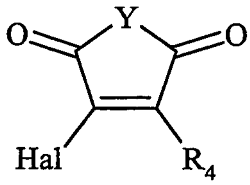

(IX)

dans laquelle Y et $R_4$ sont tels que définis dans la formule (I), et Hal représente un atome d'halogène, pour conduire aux composés de formules (I/a) et (I/b), cas particuliers des composés de formule (I), selon que T représente un groupement $R'_1$ ou G respectivement :

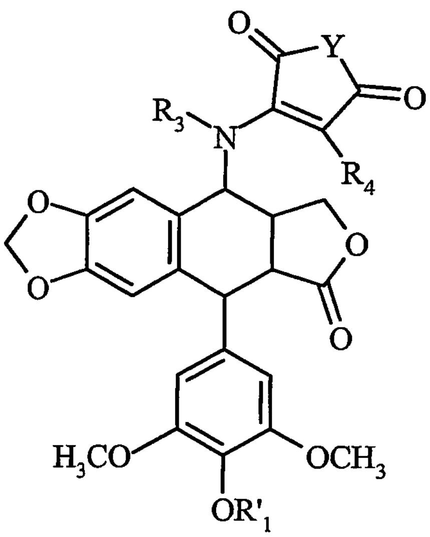

(I/a)

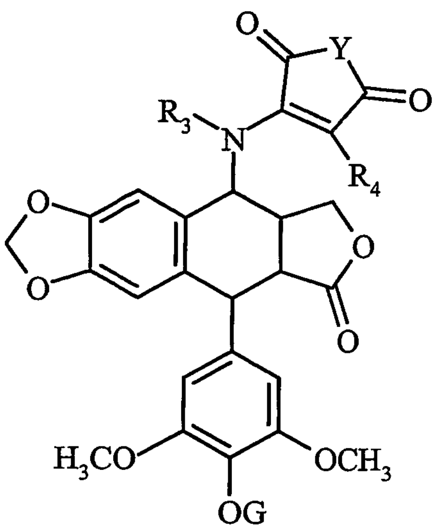

(I/b)

dans laquelle $R'_1$, $R_3$, $R_4$, Y et G sont tels que définis précédemment, composé de formule (I/b) dont on déprotège la fonction hydroxy selon les méthodes classiques de la chimie organique, pour conduire aux composés de formule (I/c), cas particuliers des composés de formule (I) :

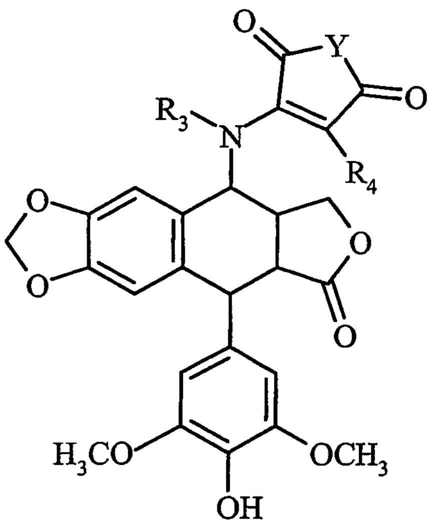

(I/c)

dans laquelle $R_3$, $R_4$ et Y sont tels que définis précédemment,

les composés (I/a) à (I/c) formant l'ensemble des composés de l'invention, que l'on purifie, le cas échéant, selon une technique classique de purification, qui peuvent, si on le désire, être séparés en leurs différents isomères optiques selon une technique classique de séparation, et que l'on transforme, si on le souhaite, en leurs sels d'addition à un acide ou à une base pharmaceutiquement acceptable.

**11.** Compositions pharmaceutiques contenant comme principe actif au moins un composé selon l'une quelconque des revendications 1 à 9, seul ou en combinaison avec un ou plusieurs excipients ou véhicules inertes, non toxiques, pharmaceutiquement acceptables.

**12.** Compositions pharmaceutiques selon la revendication 11 utile en tant que médicament, contenant au moins un principe actif selon l'une quelconque des revendications 1 à 9, utiles dans le traitement du cancer.

## Claims

**1.** Compounds of the formula (I):

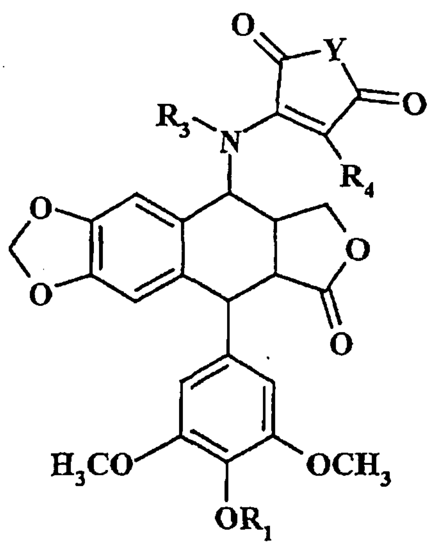

(I)

wherein:

$R_1$ represents a group chosen among hydrogen, straight or branched ($C_1$-$C_6$) alkyl, aryl, straight or branched ($C_1$-$C_6$) arylalkyl, heteroaryl, straight or branched ($C_1$-$C_6$) heteroarylalkyl, straight or branched ($C_1$-$C_6$) alkylcarbonyl, arylcarbonyl, straight or branched ($C_1$-$C_6$) arylalkylcarbonyl, straight or branched ($C_1$-$C_6$) alkoxycarbonyl, aryloxycarbonyl, straight or branched ($C_1$-$C_6$) arylalkoxycarbonyl, heterocycloalkoxycarbonyl, straight or branched ($C_1$-$C_6$) alkylsulfonyl, arylsulfonyl, straight or branched ($C_1$-$C_6$) arylalkylsulfonyl, phosphonic, or Si $(R_a)_2R_b$ wherein $R_a$ and $R_b$, identical or different, each represent a group chosen among straight or branched ($C_1$-$C_6$) alkyl, or aryl,
Y represents a group chosen among HN-NH or N-$R_2$ wherein:

$R_2$ represents a group chosen among hydrogen, straight or branched ($C_1$-$C_6$) alkyl, aryl, heteroaryl, cycloalkyl, heterocycloalkyl, straight or branched ($C_2$-$C_6$) alkenyl, straight or.branched ($C_2$-$C_6$) alkynyl, or a group of the formula -$T_1$-$R_5$ wherein:

$T_1$ represents a group chosen among a straight or branched ($C_1$-$C_6$) alkylene chain, optionally substituted by one or more groups chosen among hydroxy or straight or branched ($C_1$-$C_6$) alkoxy, a straight or branched ($C_2$-$C_6$) alkenylene chain, or a straight or branched ($C_2$-$C_6$) alkynylene chain,
$R_5$ represents a group chosen among hydroxy, straight or branched ($C_1$-$C_6$) alkoxy, straight or branched ($C_1$-$C_6$) alkylcarbonyl, straight or branched ($C_1$-$C_6$) alkylcarbonyloxy, straight or branched ($C_1$-$C_6$) alkoxycarbonyl, carboxy, halogen, trihalogenomethyl, aryl, heteroaryl, cycloalkyl, heterocycloalkyl, $NR_cR_d$ wherein $R_c$ and $R_d$, identical or different, each represent a group chosen among hydrogen, straight or branched ($C_1$-$C_6$) alkyl, straight or branched ($C_1$-$C_6$) aminoalkyl, wherein the amino part is optionally substituted by one or two identical or different groups, straight or branched ($C_1$-$C_6$) alkyl, straight or branched ($C_1$-$C_6$) hydroxyalkyl, straight or branched ($C_1$-$C_6$) alkoxy ($C_1$-$C_6$) alkyl,
or C(O)NR'$_c$R'$_d$ wherein R'$_c$ and R'$_d$, identical or different, each represent a group chosen among hydrogen, straight or branched ($C_1$-$C_6$) alkyl, straight or branched ($C_1$-$C_6$) aminoalkyl, wherein the amino part is optionally substituted by one or two identical or different groups, straight or branched ($C_1$-$C_6$) alkyl, straight or branched ($C_1$-$C_6$) hydroxyalkyl, straight or branched ($C_1$-$C_6$) alkoxy ($C_1$-$C_6$) alkyl, or R'$_c$ and R'$_d$ together form a heterocycloalkyl with the nitrogen atom which carry them,
$R_3$ represents a group chosen among hydrogen, straight or branched ($C_1$-$C_6$) alkyl, cycloalkyl, straight or branched ($C_1$-$C_6$) cycloalkylalkyl, aryl, or straight or branched ($C_1$-$C_6$) arylalkyl,
$R_4$ represents a group chosen among hydrogen, straight or branched ($C_1$-$C_6$) alkyl,

the enantiomers, diastereoisomers, and addition salts thereof to a pharmaceutically acceptable acid or base, it being understood that:

* by aryl is meant a group chosen among phenyl, biphenyl, naphthyl, dihydronaphthyl, tetrahydronaphthyl, indenyl, indanyl, and benzocyclobutyl, each of these groups optionally containing one or more substitutions, identical or different, chosen among halogen, hydroxy, straight or branched ($C_1$-$C_6$) alkyl, straight or branched ($C_1$-$C_6$) alkoxy, cyano, nitro, amino, straight or branched ($C_1$-$C_6$) alkylamino, straight or branched ($C_1$-$C_6$) dialkylamino, carboxy, straight or branched ($C_1$-$C_6$) alkoxycarbonyl, straight or branched ($C_1$-$C_6$) trihalogenoalkyl, straight or branched ($C_1$-$C_6$) alkylcarbonyloxy, straight or branched ($C_1$-$C_6$) alkylcarbonyl, and aminocarbonyl wherein the amino part is optionally substituted by one or two groups, identical or different, straight or branched ($C_1$-$C_6$) alkyl,
* by heteroaryl is meant a monocyclic or bicyclic aromatic group or a bicyclic group of which one of the rings is aromatic and the other ring is partially hydrogenated, from 5 to 12 links, containing within the cyclic system from one to three heteroatoms, identical or different, selected among oxygen, nitrogen and sulfur, the aforementioned heteroaryl group optionally being substituted by one or more identical or different groups, selected among the substituents defined previously in the case of the aryl group; among the heteroaryl groups, pyridyl, pyrrolyl, thienyl, furyl, pyrazinyl, isothiazolyl, thiazolyl, oxazolyl, isoxazolyl, pyrimidinyl, indolyl, benzofuranyl, benzothienyl, quinolyl, isoquinolyl, benzo[1,4]dioxynyl and 2,3-dihydrobenzo[1,4]dioxynyl can be cited on a purely nonrestrictive basis,
* by cycloalkyl is meant a monocyclic or bicyclic group, saturated or unsaturated but without an aromatic character, containing from 3 to 12 carbon atoms, being optionally substituted by one or more groups, identical or different, selected among halogen, straight or branched ($C_1$-$C_6$) alkyl, straight or branched ($C_1$-$C_6$) trihalogenoalkyl, hydroxy, amino, straight or branched ($C_1$-$C_6$) alkylamino, and straight or branched ($C_1$-$C_6$) dialkylamino; among the cycloalkyl groups, cyclopropyl, cyclobutyl, cyclopentyl, and

cyclohexyl can be cited on a purely nonrestrictive basis,
* by heterocycloalkyl is meant a cycloalkyl such as defined previously, containing within the cyclic system, from one to two heteroatoms, identical or different, selected among oxygen and nitrogen, the aforementioned heterocycloalkyl being optionally substituted by one or more identical or different groups defined previously in the case of the cycloalkyl group; among the heterocycloalkyl groups, piperidyl, piperazinyl, morpholyl can be cited on a purely nonrestrictive basis.

**2.** Compounds of the formula (I) according to the claim 1 wherein $R_1$ represents a hydrogen atom, the enantiomers, diastereoisomers and addition salts thereof to a pharmaceutically acceptable acid or base.

**3.** Compounds of the formula (I) according to any of the claims 1 to 2 wherein $R_3$ represents a hydrogen atom, the enantiomers, diastereoisomers and addition salts thereof to a pharmaceutically acceptable acid or base.

**4.** Compounds of the formula (I) according to any of the claims 1 to 3 wherein $R_4$ represents a hydrogen atom or a methyl group, the enantiomers, diastereoisomers and addition salts thereof to a pharmaceutically acceptable acid or base.

**5.** Compounds of the formula (I) according to any of the claims 1 to 4 wherein Y represents a HN-NH or N-$R_2$ group wherein $R_2$ represents a straight or branched ($C_1$-$C_6$) alkyl group, straight or branched ($C_2$-$C_6$) alkenyl group, or a group of the formula -$T_1$-$R_5$ wherein $T_1$ and $R_5$ are such as defined in the formula (I), the enantiomers, diastereoisomers and addition salts thereof to a pharmaceutically acceptable acid or base.

**6.** Compounds of the formula (I) according to any of the claims 1 to 5 wherein Y represents a group of the formula $NR_2$ wherein $R_2$ represents a methyl group, the enantiomers, diastereoisomers and addition salts thereof to a pharmaceutically acceptable acid or base.

**7.** Compounds of the formula (I) according to any of the claims 1 to 6 wherein Y represents a group of the formula $NR_2$ wherein $R_2$ represents a -$T_1$-$R_6$ group wherein $T_1$ represents a straight or branched ($C_1$-$C_6$) alkylene chain, and $R_5$ represents a group chosen among aryl, carboxy and straight or branched ($C_1$-$C_6$) alkylcarbonyloxy, the enantiomers, diastereoisomers and addition salts thereof to a pharmaceutically acceptable acid or base.

**8.** Compounds of the formula (I) according to any of the claims 1 to 7 wherein Y represents a group of the formula $NR_2$ wherein $R_2$ represents a -$T_1$-$R_5$ group wherein $T_1$ represents a methylene -$CH_2$- group and $R_5$ represents an aryl group, the enantiomers, diastereoisomers and addition salts thereof to a pharmaceutically acceptable acid or base.

**9.** Compounds of the formula (I) according to the claim 1 which are:

- 3-{[(5*S*,5a*S*,8a*R*,9*R*)-9-(4-hydroxy-3,5-dimethoxyphenyl)-8-oxo-5,5a,6,8,8a,9-hexahydrofuro[3',4':6,7]naphtho[2,3-*d*][1,3]dioxol-5-yl]amino}-1-methyl-1*H*-pyrrole-2,5-dione;
- 3-{[(5*S*,5a*S*,8a*R*,9*R*)-9-(4-hydroxy-3,5-dimethoxyphenyl)-8-oxo-5,5a,6,8,8a,9-hexahydrofuro[3',4':6,7]naphtho[2,3-*d*][1,3]dioxol-5-yl]amino}-1-benzyl-1*H*-pyrrole-2,5-dione;
- 3-([(5*S*,5a*S*,8a*R*,9*R*)-9-(4-hydroxy-3,5-dimethoxyphenyl)-8-oxo-5,5a,6,8,8a,9-hexahydrofuro[3',4':6,7]naphtho[2,3-*d*][1,3]dioxol-5-yl]amino}-1-(4-(fluorobenzyl)-1*H*-pyrrole-2,5-dione;
- 3-{[(5*S*,5a*S*,8a*R*,9*R*)-9-(4-hydroxy-3,5-dimeth-oxyphenyl)-8-oxo-5,5a,6,8,8a,9-hexahydrofuro[3',4':6,7]naphtho[2,3-*d*][1,3]dioxol-5-yl]amino}-1-[4-(trifluoromethyl)benzyl]-1*H*-pyrrole-2,5-dione;
- *N*-{4-[(3-{[(5*S*,5a*S*,8a*R*,9*R*)-9-(4-hydroxy-3,5-dimethoxyphenyl)-8-oxo-5,5a,6,8,8a,9-hexahydrofuro[3',4':6,7]naphtho[2,3-*d*][1,3]dioxol-5-yl]amino}-2,5-dioxo-2,5-dihydro-1*H*-pyrrol-1-yl)methyl]phenyl}acetamide;
- 6-(3-{[(5*S*,5a*S*,8a*R*,9*R*)-9-(4-hydroxy-3,5-dimethoxyphenyl)-8-oxo-5,5a,6,8,8a,9-hexahydrofuro[3',4':6,7]naphtho[2,3-*d*][1,3]dioxol-5-yl]amino}-2,5-dioxo-2,5-dihydro-1*H*-pyrrol-1-yl) hexanoic acid;
- 3-{[(5*S*,5a*S*,8a*R*,9*R*)-9-(4-hydroxy-3,5-dimethoxyphenyl)-8-oxo-5,5a,6,8,8a,9-hexahydrofuro[3',4':6,7]naphtho[2,3-*d*][1,3]dioxol-5-yl]amino}-1-butyl-1*H*-pyrrole-2,5-dione;
- 3-{[(5*S*,5a*S*,8a*R*,9*R*)-9-(4-hydroxy-3,5-dimethoxyphenyl)-8-oxo-5,5a,6,8,8a,9-hexahydrofuro[3',4':6,7]naphtho[2,3-*d*][1,3]dioxol-5-yl]amino}-1-allyl-1*H*-pyrrole-2,5-dione;
- 2-(3-{[(5*S*,5a*S*,8a*R*,9*R*)-9-(4-hydroxy-3,5-dimethoxyphenyl)-8-oxo-5,5a,6,8,8a,9-hexahydrofuro[3',4':6,7]naphtho[2,3-*d*][1,3]dioxol-5-yl]amino}-2,5-dioxo-2,5-dihydro-1*H*-pyrrol-1-yl) ethyl acetate;
- 3-{[(5*S*,5a*S*,8a*R*,9*R*)-9-(4-hydroxy-3,5-dimethoxyphenyl)-8-oxo-5,5a,6,8,8a,9-hexahydrofuro[3.',4':6,7]naphtho[2,3-*d*][1,3]dioxol-5-yl]amino}-1-(2,3-dihydroxypropyl)-1*H*-pyrrole-2,5-dione;

- 3-{[(5*S*,5a*S*,8a*R*,9*R*)-9-(4-hydroxy-3,5-dimethoxyphenyl)-8-oxo-5,5a,6,8,8a,9-hexahydrofuro[3',4':6,7]naphtho[2,3-*d*][1,3]dioxol-5-yl]amino}-1-[2-(dimethylamino)ethyl]-1*H*-pyrrole-2,5-dione.

**10.** A method for the preparation of the compounds of the formula (I), wherein is used as starting product a compound of the formula (II):

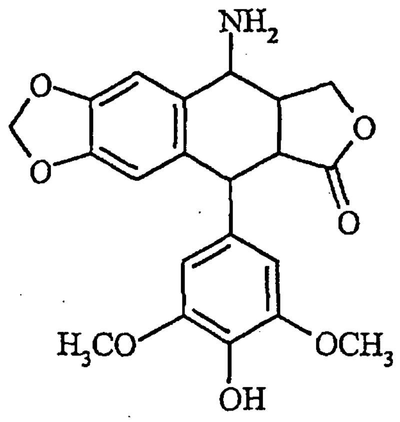

(II)

which is subjected, under basic conditions:

- either to the action of a compound of the formula (III) :

$R'_1$-X (III)

wherein $R'_1$ represents a group chosen among straight or branched ($C_1$-$C_6$) alkyl, aryl, straight or branched ($C_1$-$C_6$) arylalkyl, heteroaryl, straight or branched ($C_1$-$C_6$) heteroarylalkyl, straight or branched ($C_1$-$C_6$) alkylcarbonyl, arylcarbonyl, straight or branched ($C_1$-$C_6$) arylalkylcarbonyl, straight or branched ($C_1$-$C_6$) alkoxycarbonyl, aryloxycarbonyl, straight or branched ($C_1$-$C_6$) arylalkoxycarbonyl, heterocycloalkoxycarbonyl, straight or branched ($C_1$-$C_6$) alkylsulfonyl, arylsulfonyl, straight or branched ($C_1$-$C_6$) arylalkylsulfonyl, phosphonic, or $Si(R_a)_2R_b$ wherein $R_a$ and $R_b$, identical or different, each represent a group chosen among straight or branched ($C_1$-$C_6$) alkyl, or aryl, and X represents a hydrogen atom, a halogen atom or an ordinary leaving group of organic chemistry, to lead to the compounds of the formula (IV/a) :

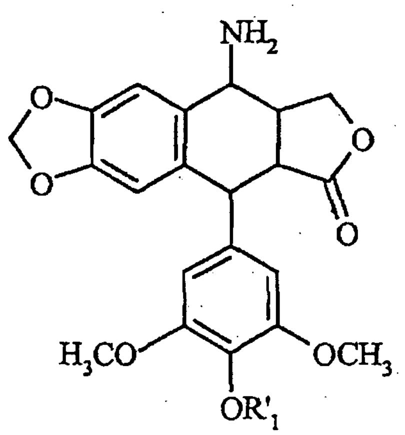

(IV/a)

wherein $R'_1$ is such as defined previously,

- or to the action of a compound of the formula (V):

G-L (V)

wherein G represents a traditional protective group of hydroxy functions and L an ordinary leaving group of organic chemistry, to lead to the compounds of the formula (IV/b):

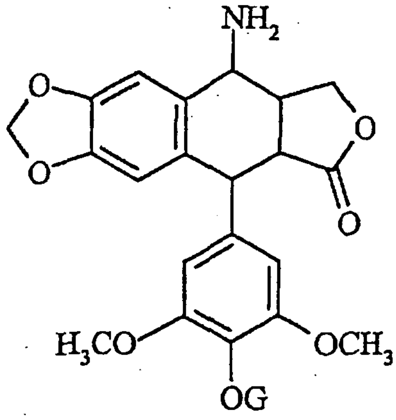

(IV/b)

wherein G is such as defined previously,
the whole of the compounds of the formula (IV/a) and (IV/b) forming the compounds of the formula (IV):

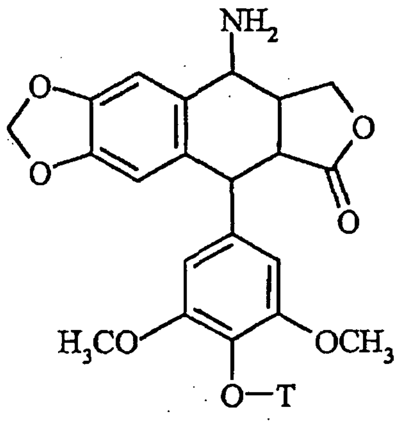

(IV)

wherein T represents an $R'_1$ group or G such as previously defined,
a compound of the formula (IV), which is subjected, under basic conditions, to the action of a compound of the formula (VI) :

$R'_3$-X' (VI)

wherein $R'_3$ represents a group chosen among straight or branched ($C_1$-$C_6$) alkyl, cycloalkyl, straight or branched ($C_1$-$C_6$) cycloalkylalkyl, aryl or straight or branched ($C_1$-$C_6$) arylalkyl,
and X' represents a hydrogen atom, a halogen atom or an ordinary leaving group of organic chemistry, to lead to the compounds of the formula (VII):

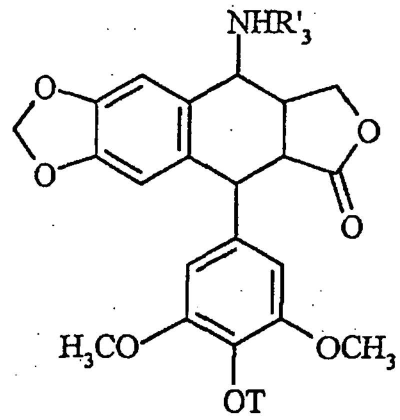

(VII)

wherein $R'_3$ and T are such as previously defined,
the whole of the compounds of the formulas (IV) and (VII) forming the compounds of the formula (VIII):

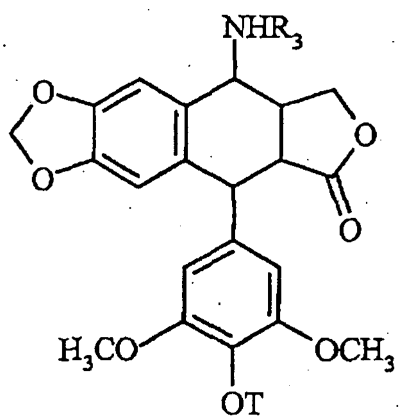

(VIII)

wherein $R_3$ and T are such as defined in the formula (I),
a compound of the formula (VIII) which are treated in a basic medium by a compound of the formula (IX) :

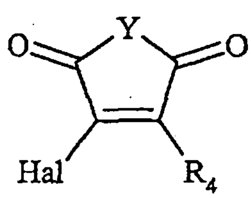

(IX)

wherein Y and $R_4$ are such as defined in the formula (I),
and Hal represents a halogen atom, to lead to the compounds of the formulas (I/a) and (I/b), specific cases of the compounds of the formula (I), according to whether T represents an $R'_1$ group or G, respectively:

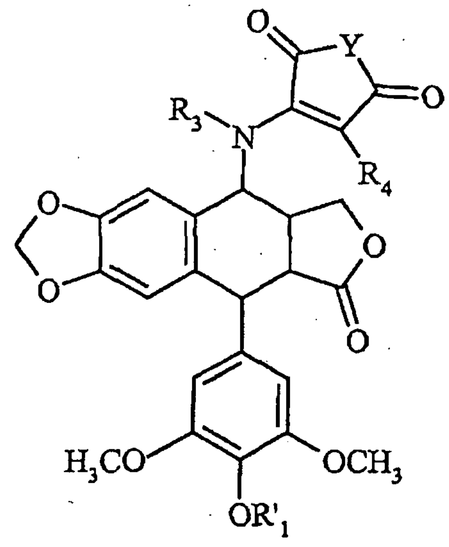

(I/a)

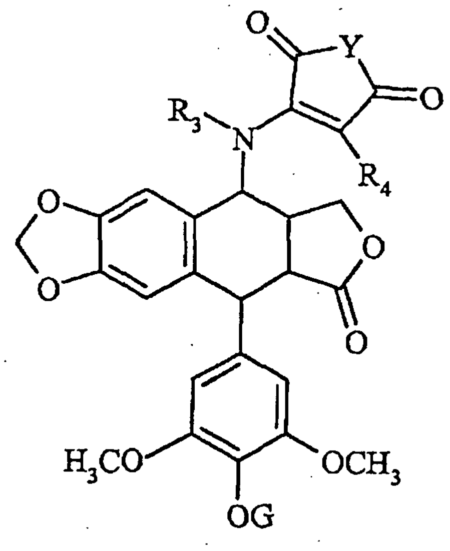

(I/b)

wherein $R'_1$, $R_3$, $R_4$, Y and G are such as previously defined,
a compound of the formula (I/b) wherein the hydroxy function is deprotected according to the traditional methods of organic chemistry, to lead to the compounds of the formula (I/c), specific cases of the compounds of the formula (I):

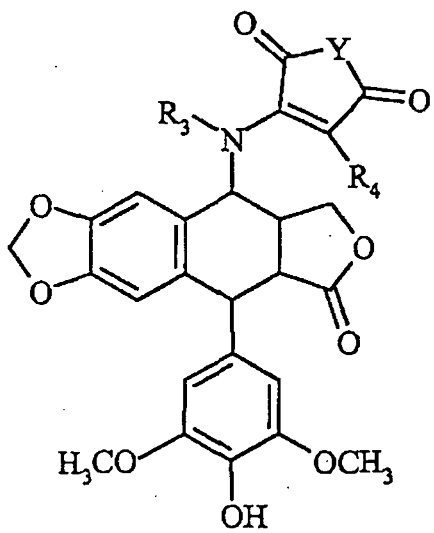

(I/c)

wherein $R_3$, $R_4$ and Y are such as previously defined,
the compounds (I/a) to (I/c) form the whole of the compounds of the invention, which can be purified, if necessary, according to a traditional purification technique, which can, if it is desired, be separated into the various optical isomers thereof according to a traditional separation technique, and which can be transformed, if it is desired, into the addition salts thereof to a pharmaceutically acceptable acid or base.

**11.** Pharmaceutical compositions containing as an active ingredient at least one compound according to any of the claims 1 to 9, alone or in combination with one or more nontoxic, inert, pharmaceutically acceptable excipients or vehicles.

12. Pharmaceutical compositions according to the claim 11 useful as a drug, containing at least one active ingredient according to any of the claims 1 to 9, useful in the treatment of cancer.

**Patentansprüche**

1. Verbindungen der Formel (I):

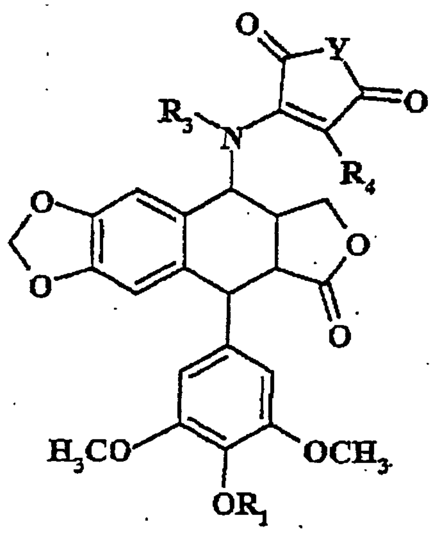

(I)

in welcher:

$R_1$ für eine Gruppe steht, welche ausgewählt wird unter Wasserstoff, linearem oder verzweigtem ($C_1$-$C_6$)-Alkyl, Aryl, Aryl-lineares oder verzweigtes ($C_1$-$C_6$)-alkyl, Heteroaryl, Heteroaryl-lineares oder verzweigtes ($C_1$-$C_6$)-alkyl, linearem oder verzweigtem ($C_1$-$C_6$)-Alkylcarbonyl, Arylcarbonyl, Aryl-lineares oder verzweigtes ($C_1$-$C_6$)-alkylcarbonyl, linearem oder verzweigtem ($C_1$-$C_6$)-Alkoxycarbonyl, Aryloxycarbonyl, Aryl-lineares oder verzweigtes ($C_1$-$C_6$)-alkoxycarbonyl, Heterocycloalkoxycarbonyl, linearem oder verzweigtem ($C_1$-$C_6$)-Alkylsulfonyl, Arylsulfonyl, Aryl-lineares oder verzweigtes ($C_1$-$C_6$)-alkylsulfonyl, Phosphonat oder $Si(R_a)_2R_b$, in welchem $R_a$, $R_b$, die gleich oder verschieden sind, jeweils für eine Gruppe, welche unter linearem oder verzweigtem ($C_1$-$C_6$)-Alkyl oder Aryl ausgewählt wird, stehen,
Y für eine unter HN-NH oder N-$R_2$ ausgewählte Gruppe steht, in welcher:

$R_2$ für eine Gruppe steht, welche ausgewählt wird unter Wasserstoff, linearem oder verzweigtem ($C_1$-$C_6$)-Alkyl, Aryl, Heteroaryl, Cycloalkyl, Heterocycloalkyl, linearem oder verzweigtem ($C_2$-$C_6$)-Alkenyl, linearem oder verzweigtem ($C_2$-$C_6$)-Alkinyl oder einer Gruppe mit der Formel -$T_1$-$R_5$, in welcher:

♦ $T_1$ für eine Gruppe steht, welche ausgewählt wird unter einer linearen oder verzweigten ($C_1$-$C_6$)-Alkylenkette, welche gegebenenfalls substituiert ist durch eine oder mehrere Gruppen, welche ausgewählt werden unter Hydroxy oder linearem oder verzweigtem ($C_1$-$C_6$)-Alkoxy, einer linearen oder verzweigten ($C_2$-$C_6$)-Alkenylenkette oder einer linearen oder verzweigten ($C_2$-$C_6$)-Alkinylenkette,
♦ $R_5$ für eine Gruppe steht, welche ausgewählt wird unter Hydroxy, linearem oder verzweigtem ($C_1$-$C_6$)-Alkoxy, linearem oder verzweigtem ($C_1$-$C_6$)-Alkylcarbonyl, linearem oder verzweigtem ($C_1$-$C_6$)-Alkylcarbonyloxy, linearem oder verzweigtem ($C_1$-$C_6$)-Alkoxycarbonyl, Carboxy, Halogen, Trihalogenmethyl, Aryl, Heteroaryl, Cycloalkyl, Heterocycloalkyl, $NR_cR_d$, in welchem $R_c$, $R_d$, die gleich oder verschieden sind, jeweils für eine Gruppe stehen, welche ausgewählt wird unter Wasserstoff, linearem oder verzweigtem ($C_1$-$C_6$)-Alkyl, linearem oder verzweigtem ($C_1$-$C_6$)-Aminoalkyl, in welchem der Amino-Anteil gegebenenfalls durch eine oder zwei identische oder unterschiedliche Gruppen, lineares oder verzweigtes ($C_1$-$C_6$)-Alkyl, lineares oder verzweigtes ($C_1$-$C_6$)-Hydroxyalkyl, lineares oder verzweigtes ($C_1$-$C_6$)-Alkoxy-($C_1$-$C_6$)-alkyl substituiert ist,

oder $C(O)NR'_cR'_d$, in welchem $R'_c$, $R'_d$, welche gleich oder verschieden sind, jeweils für eine Gruppe stehen,

welche ausgewählt wird unter Wasserstoff, linearem oder verzweigtem ($C_1$-$C_6$)-Alkyl, linearem oder verzweigtem ($C_1$-$C_6$)-Aminoalkyl, in welchem der Amino-Anteil gegebenenfalls durch eine oder zwei identische oder unterschiedliche Gruppen, lineares oder verzweigtes ($C_1$-$C_6$)-Alkyl, lineares oder verzweigtes ($C_1$-$C_6$)-Hydroxyalkyl, lineares oder verzweigtes ($C_1$-$C_6$)-Alkoxy-($C_1$-$C_6$)-alkyl substituiert ist, oder $R'_c$ und $R'_d$ zusammen mit dem Stickstoffatom, welches diese trägt, ein Heterocycloalkyl bilden,

$R_3$ für eine Gruppe steht, welche unter Wasserstoff, linearem oder verzweigtem ($C_1$-$C_6$)-Alkyl, Cycloalkyl, Cycloalkyl-lineares oder verzweigtes ($C_1$-$C_6$)-alkyl, Aryl oder Aryl-lineares oder verzweigtes ($C_1$-$C_6$)-alkyl ausgewählt wird,
$R_4$ für eine Gruppe steht, welche unter Wasserstoff, linearem oder verzweigtem ($C_1$-$C_6$)-Alkyl ausgewählt wird,

deren Enantiomere, Diastereomere wie auch deren Additionssalze mit einer pharmazeutisch annehmbaren Säure oder Base,
wobei es sich versteht, dass:

* *unter Aryl* eine Gruppe verstanden wird, welche ausgewählt wird unter Phenyl, Biphenyl, Naphthyl, Dihydronaphthyl, Tetrahydronaphthyl, Indenyl, Indanyl und Benzocyclobutyl, wobei jede dieser Gruppierungen gegebenenfalls eine oder mehrere Substitutionen, die gleich oder verschieden sind, umfasst, welche ausgewählt werden unter Halogen, Hydroxy, linearem oder verzweigtem ($C_1$-$C_6$)-Alkyl, linearem oder verzweigtem ($C_1$-$C_6$)-Alkoxy, Cyan, Nitro, Amino, linearem oder verzweigtem ($C_1$-$C_6$)-Alkylamino, linearem oder verzweigtem ($C_1$-$C_6$)-Dialkylamino, Carboxy, linearem oder verzweigtem ($C_1$-$C_6$)-Alkoxycarbonyl, linearem oder verzweigtem Trihalogen-($C_1$-$C_6$)-alkyl, linearem oder verzweigtem ($C_1$-$C_6$)-Alkylcarbonyloxy, linearem oder verzweigtem ($C_1$-$C_6$)-Alkylcarbonyl und Aminocarbonyl, in welchem der Amino-Anteil gegebenenfalls durch eine oder zwei Gruppierungen, die gleich oder verschieden sind, lineares oder verzweigtes ($C_1$-$C_6$)-Alkyl, substituiert ist,
* *unter Heteroaryl* eine mono- oder bicyclische aromatische Gruppe oder eine bicyclische Gruppe, bei welcher einer der Cyclen aromatisch ist und der andere Cyclus partiell hydriert ist, mit 5 bis 12 Ringatomen, welche innerhalb des cyclischen Systems ein bis drei Heteroatome, die gleich oder verschieden sind, ausgewählt unter Sauerstoff, Stickstoff und Schwefel, umfassen, verstanden wird, wobei die Heteroarylgruppe gegebenenfalls durch eine oder mehrere Gruppierungen, die gleich oder verschieden sind, die unter den zuvor in dem Falle der Arylgruppe definierten Substituenten ausgewählt werden, substituiert sein kann; unter den Heteroarylgruppen kann man nicht-einschränkend die Pyridyl-, Pyrrolyl-, Thienyl-, Furyl-, Pyrazinyl-, Isothiazolyl-, Thiazolyl-, Oxazolyl-, Isoxazolyl-, Pyrimidinyl-, Indolyl-, Benzofuranyl-, Benzothienyl-, Chinolyl-, Isochinolyl-, Benzo-[1,4]-dioxinyl-, 2,3-Dihydrobenzo-[1,4]-dioxinylgruppe aufführen,
* *unter Cycloalkyl* eine gesättigte oder ungesättigte, aber keinen aromatischen Charakter aufweisende mono- oder bicyclische Gruppe verstanden wird, die 3 bis 12 Kohlenstoffatome umfasst, welche gegebenenfalls durch eine oder mehrere Gruppierungen, die gleich oder verschieden sind, die ausgewählt werden unter Halogen, linearem oder verzweigtem ($C_1$-$C_6$)-Alkyl, linearem oder verzweigtem Trihalogen-($C_1$-$C_6$)-alkyl, Hydroxy, Amino, linearem oder verzweigtem ($C_1$-$C_6$)-Alkylamino und linearem oder verzweigtem ($C_1$-$C_6$)-Dialkylamino, substituiert ist; unter den Cycloalkylgruppen kann man nicht-einschränkend die Cyclopropyl-, Cyclobutyl-, Cyclopentyl-, Cyclohexylgruppe aufführen,
* *unter Heterocycloalkyl* ein Cycloalkyl, wie zuvor definiert, verstanden wird, welches innerhalb des cyclischen Systems ein bis zwei Heteroatome, die gleich oder verschieden sind, welche unter Sauerstoff und Stickstoff ausgewählt werden, umfasst, wobei das Heterocycloalkyl gegebenenfalls substituiert ist durch eine oder mehrere gleiche oder unterschiedliche Gruppierungen, die zuvor in dem Falle der Cycloalkylgruppe definiert worden sind; unter den Heterocycloalkylgruppen kann man nicht-einschränkend die Piperidyl-, Piperazinyl-, Morpholylgruppe aufführen.

**2.** Verbindungen der Formel (I) nach Anspruch 1, **dadurch gekennzeichnet, dass** $R_1$ für ein Wasserstoffatom steht, deren Enantiomere, Diastereomere wie auch deren Additionssalze mit einer pharmazeutisch annehmbaren Säure oder Base.

**3.** Verbindungen der Formel (I) nach einem der Ansprüche 1 bis 2, **dadurch gekennzeichnet, dass** $R_3$ für ein Wasserstoffatom steht, deren Enantiomere, Diastereomere wie auch deren Additionssalze mit einer pharmazeutisch annehmbaren Säure oder Base.

**4.** Verbindungen der Formel (I) nach einem der Ansprüche 1 bis 3, **dadurch gekennzeichnet, dass** $R_4$ für ein Wasserstoffatom oder eine Methylgruppe steht, deren Enantiomere, Diastereomere wie auch deren Additionssalze mit einer pharmazeutisch annehmbaren Säure oder Base.

5. Verbindungen der Formel (I) nach einem der Ansprüche 1 bis 4, **dadurch gekennzeichnet, dass** Y für eine Gruppe HN-NH oder N-$R_2$, in welcher $R_2$ für eine lineare oder verzweigte ($C_1$-$C_6$)-Alkylgruppe, lineare oder verzweigte ($C_2$-$C_6$)-Alkenylgruppe oder eine Gruppe der Formel -$T_1$-$R_5$, in welcher $T_1$ und $R_5$ so wie in der Formel (I) definiert sind, steht, steht, deren Enantiomere, Diastereomere wie auch deren Additionssalze mit einer pharmazeutisch annehmbaren Säure oder Base.

6. Verbindungen der Formel (I) nach einem der Ansprüche 1 bis 5, **dadurch gekennzeichnet, dass** Y für eine Gruppe der Formel $NR_2$ steht, in welcher $R_2$ für eine Methylgruppe steht, deren Enantiomere, Diastereomere wie auch deren Additionssalze mit einer pharmazeutisch annehmbaren Säure oder Base.

7. Verbindungen der Formel (I) nach einem der Ansprüche 1 bis 6, **dadurch gekennzeichnet, dass** Y für eine Gruppe der Formel $NR_2$ steht, in welcher $R_2$ für eine Gruppe $T_1$-$R_5$ steht, in welcher $T_1$ für eine lineare oder verzweigte ($C_1$-$C_6$)-Alkylenkette steht und $R_5$ für eine unter Aryl, Carboxy und linearem oder verzweigtem ($C_1$-$C_6$)-Alkylcarbonyloxy ausgewählte Gruppe steht, deren Enantiomere, Diastereomere wie auch deren Additionssalze mit einer pharmazeutisch annehmbaren Säure oder Base.

8. Verbindungen der Formel (I) nach einem der Ansprüche 1 bis 7, **dadurch gekennzeichnet, dass** Y für eine Gruppe der Formel $NR_2$ steht, in welcher $R_2$ für eine Gruppe $T_1$-$R_5$ steht, in welcher $T_1$ für eine Methylengruppe -$CH_2$- steht und $R_5$ für eine Arylgruppe steht, deren Enantiomere, Diastereomere wie auch deren Additionssalze mit einer pharmazeutisch annehmbaren Säure oder Base.

9. Verbindungen der Formel (I) nach Anspruch 1, die sind:

- 3-{[(5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho-[2,3-*d*][1,3]dioxol-5-yl]amino}-1-methyl-1*H*-pyrrol-2,5-dion;
- 3-{[(5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho-[2,3-*d*][1,3]dioxol-5-yl]amino}-1-benzyl-1*H*-pyrrol-2,5-dion;
- 3-{[(5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho-[2,3-*d*][1,3]dioxol-5-yl]amino}-1-(4-fluorbenzyl)-1*H*-pyrrol-2,5-dion;
- 3-{[(5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho-[2,3-*d*][1,3]dioxol-5-yl]amino}-1-[4-(trifluormethyl)benzyl]-1*H*-pyrrol-2,5-dion;
- *N*-{4-[(3-{[(5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho-[2,3-*d*][1,3]dioxol-5-yl]amino}-2,5-dioxo-2,5-dihydro-1*H*-pyrrol-1-yl)-methyl]phenyl}acetamid;
- 6-(3-{[(5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho-[2,3-*d*][1,3]dioxol-5-yl]amino}-2,5-dioxo-2,5-dihydro-1*H*-pyrrol-1-yl)-hexansäure;
- 3-{[(5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho-[2,3-*d*][1,3]dioxol-5-yl]amino}-1-butyl-1*H*-pyrrol-2,5-dion;
- 3-{[(5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho-[2,3-*d*][1,3]dioxol-5-yl]amino}-1-allyl-1*H*-pyrrol-2,5-dion;
- 2-(3-{[(5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho-[2,3-*d*][1,3]dioxol-5-yl]amino}-2,5-dioxo-2,5-dihydro-1*H*-pyrrol-1-yl)-ethylacetat;
- 3-{[(5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho-[2,3-*d*][1,3]dioxol-5-yl]amino}-1-(2,3-dihydroxypropyl)-1*H*-pyrrol-2,5-dion;
- 3-{[(5*S*,5a*S*,8a*R*,9*R*)-9-(4-Hydroxy-3,5-dimethoxyphenyl)-8-oxo-5,5a,6,8,8a,9-hexahydrofuro[3',4':6,7]naphtho-[2,3-*d*][1,3]dioxol-5-yl]amino}-1-[2-(dimethylamino)ethyl]-1*H*-pyrrol-2,5-dion.

10. Verfahren zur Herstellung der Verbindungen der Formel (I), **dadurch gekennzeichnet, dass** man als Ausgangsprodukt eine Verbindung der Formel (II) einsetzt:

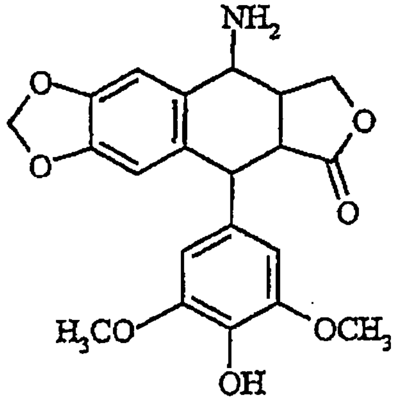

(II)

,

welche unter basischen Bedingungen unterworfen wird:

➢ entweder der Wirkung einer Verbindung der Formel (III):

$R'_1$ - X (III),

in welcher $R'_1$ für eine Gruppe steht, welche unter linearem oder verzweigtem ($C_1$-$C_6$)-Alkyl, Aryl, Aryl-lineares oder verzweigtes ($C_1$-$C_6$)-alkyl, Heteroaryl, Heteroaryl-lineares oder verzweigtes ($C_1$-$C_6$)-alkyl, linearem oder verzweigtem ($C_1$-$C_6$)-Alkylcarbonyl, Arylcarbonyl, Aryl-lineares oder verzweigtes ($C_1$-$C_6$)-alkylcarbonyl, linearem oder verzweigtem ($C_1$-$C_6$)-Alkoxycarbonyl, Aryloxycarbonyl, Aryl-lineares oder verzweigtes ($C_1$-$C_6$)-alkoxycarbonyl, Heterocycloalkoxycarbonyl, linearem oder verzweigtem ($C_1$-$C_6$)-Alkylsulfonyl, Arylsulfonyl, Aryl-lineares oder verzweigtes ($C_1$-$C_6$)-alkylsulfonyl, Phosphonat oder $Si(R_a)_2R_b$, in welchem $R_a$, $R_b$, die gleich oder verschieden sind, jeweils für eine Gruppe, welche unter linearem oder verzweigtem ($C_1$-$C_6$)-Alkyl oder Aryl ausgewählt wird, stehen, ausgewählt wird,
und X für ein Wasserstoffatom, ein Halogenatom oder eine übliche austretende Gruppe der organischen Chemie steht, um zu den Verbindungen der Formel (IV/a) zu gelangen:

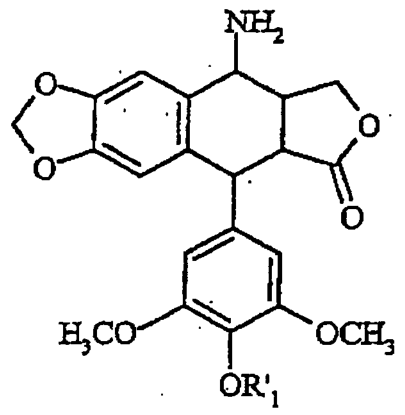

(IV/a)

,

in welcher $R'_1$ wie zuvor definiert ist,
➢ oder der Wirkung einer Verbindung der Formel (V):

G - L (V),

in welcher G für eine klassische Schutzgruppe der Hydroxyfunktionen steht und L für eine übliche austretende Gruppe der organischen Chemie steht, um zu den Verbindungen der Formel (IV/b) zu gelangen:

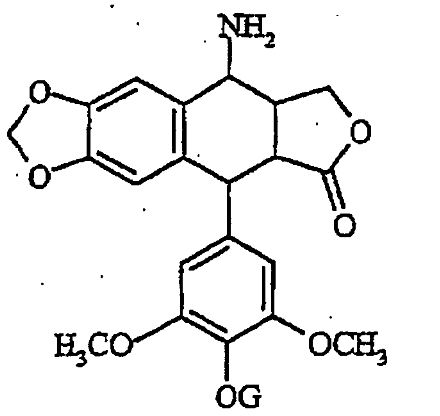

(IV/b)

,

in welcher G wie zuvor definiert ist,
wobei die Gesamtheit der Verbindungen der Formel (IV/a) und (IV/b) die Verbindungen der Formel (IV) bildet:

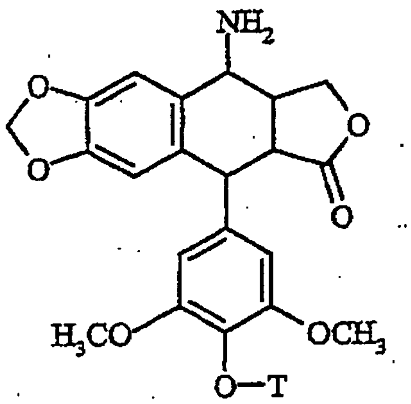

(IV)

,

in welcher T für eine Gruppe $R'_1$ oder G, wie sie zuvor definiert wurden, steht,
wobei die Verbindung der Formel (IV) unter basischen Bedingungen der Wirkung einer Verbindung der Formel (VI) unterworfen wird:

$R'_3$ - X' (VI),

in welcher $R'_3$ für eine Gruppe steht, welche unter linearem oder verzweigtem ($C_1$-$C_6$)-Alkyl, Cycloalkyl, Cycloalkyl-lineares oder verzweigtes ($C_1$-$C_6$)-alkyl, Aryl oder Aryl-lineares oder verzweigtes ($C_1$-$C_6$)-alkyl ausgewählt wird,
und X' für ein Wasserstoffatom, ein Halogenatom oder eine übliche austretende Gruppe der organischen Chemie steht, um zu den Verbindungen der Formel (VII) zu gelangen:

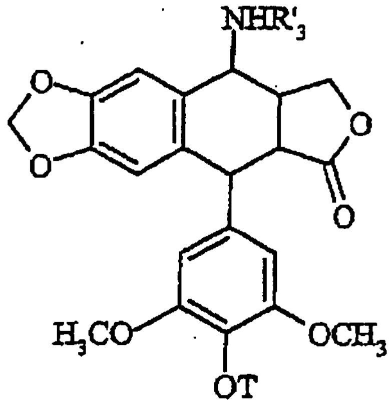

(VII)

,

in welcher $R'_3$ und T wie zuvor definiert sind,
wobei die Gesamtheit der Verbindungen (IV) und (VII) die Verbindungen der Formel (VIII) bildet:

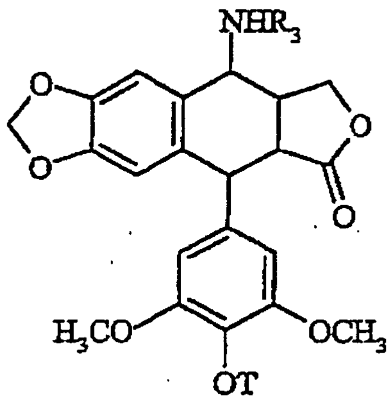

(VIII)

,

in welcher $R_3$ und T wie in der Formel (I) definiert sind,
wobei man die Verbindung der Formel (VIII) in basischem Milieu mit einer Verbindung der Formel (IX) behandelt:

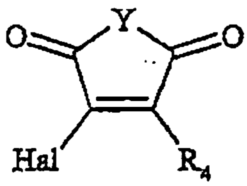

(IX)

,

in welcher Y und $R_4$ wie in der Formel (I) definiert sind,
und Hal für ein Halogenatom steht, um zu den Verbindungen der Formeln (I/a) und (I/b) zu gelangen, besonderen Fällen der Verbindungen der Formel (I), gemäß welcher T für eine Gruppe $R'_1$ bzw. G steht:

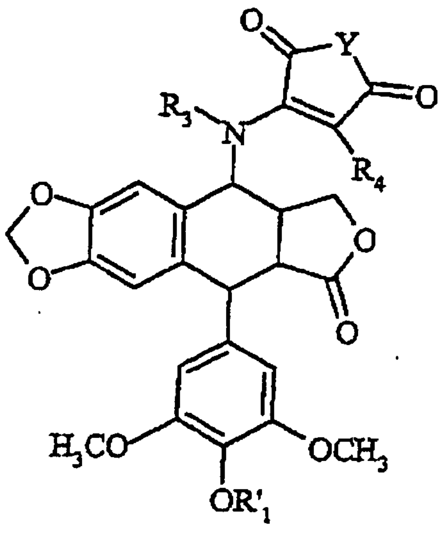

(I/a)

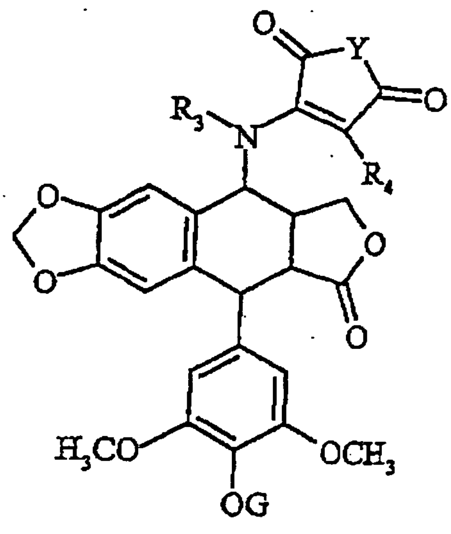

(I/b) ,

in welcher $R'_1$, $R_3$, $R_4$, Y und G wie zuvor definiert sind,
von welcher Verbindung der Formel (I/b) man die Schutzgruppe von der Hydroxyfunktion gemäß den klassischen Methoden der organischen Chemie entfernt, um zu den Verbindungen der Formel (I/c) zu gelangen, besonderen Fällen der Verbindungen der Formel (I):

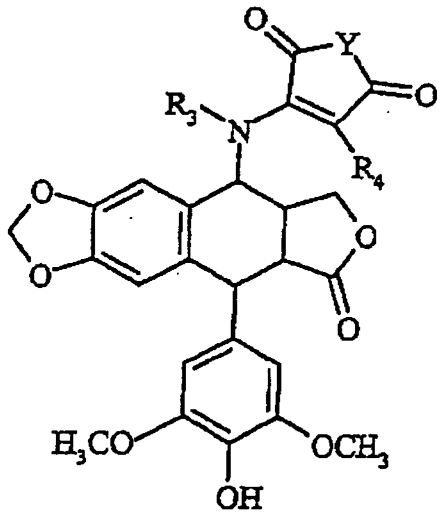

(I/c) ,

in welcher $R_3$, $R_4$ und Y wie zuvor definiert sind,

wobei die Verbindungen (I/a) bis (I/c) die Gesamtheit der Verbindungen der Erfindung bilden, die man gegebenenfalls gemäß einer klassischen Reinigungstechnik reinigt, die, wenn man dies wünscht, in ihre verschiedenen optischen Isomere gemäß einer klassischen Trenntechnik getrennt werden können und die man, wenn man dies wünscht, in deren Additionssalze mit einer pharmazeutisch annehmbaren Säure oder Base umwandelt.

**11.** Pharmazeutische Zusammensetzungen, die als Wirkstoff wenigstens eine Verbindung nach einem der Ansprüche 1 bis 9 allein oder in Kombination mit einem oder mehreren inerten, nicht-toxischen, pharmazeutisch annehmbaren Trägem oder Vehikeln enthalten.

**12.** Pharmazeutische Zusammensetzungen nach Anspruch 11, welche als Arzneimittel nützlich sind, welche wenigstens einen Wirkstoff nach einem der Ansprüche 1 bis 9 enthalten, welche bei der Behandlung von Krebs nützlich sind.